# EUROPEAN PATENT SPECIFICATION

(11) **EP 4 100 715 B1**
(45) Date of publication and mention of the grant of the patent: **27.05.2026**
(21) Application number: 21707393.1
(22) Date of filing: 05.02.2021
(51) Int. Cl.: G01N 33/50, G01N 33/80, G01N 15/14, C12Q 1/6881, G01N 33/53, G01N 15/01

(54) **PARTICLE ANALYSIS METHOD AND PARTICLE ANALYZER**
TEILCHENANALYSEVERFAHREN UND TEILCHENANALYSATOR
PROCÉDÉ D'ANALYSE DE PARTICULES ET ANALYSEUR DE PARTICULES

(30) Priority: 05.02.2020 JP 2020018264
(43) Date of publication of application: 14.12.2022
(73) Proprietor: Nihon Kohden Corporation, Shinjuku-ku Tokyo 161-8560 (JP)
(72) Inventor: NAGAI, Yutaka, Tokorozawa-shi, Saitama 359-0037 (JP)
(74) Representative: Haseltine Lake Kempner LLP
(86) International application number: PCT/JP2021/004376
(87) International publication number: WO 2021/157710

(56) References cited:
- US-A1- 2009 130 647
- BRIAN T. GRIMBERG: "Methodology and application of flow cytometry for investigation of human malaria parasites", JOURNAL OF IMMUNOLOGICAL METHODS, vol. 367, no. 1-2, 1 March 2011 (2011-03-01), pages 1 - 16, XP055035863, ISSN: 0022-1759, DOI: 10.1016/j.jim.2011.01.015

## Description

### Technical Field

The present invention relates to a particle analysis method and a particle analyzer.

### Background Art

Conventionally, particles contained in a blood sample have been classified and counted. For example, in an optical automatic blood cell analyzer, a flow cytometer is used to analyze the particles in the blood sample. In a flow cytometry method, specifically, information such as an amount of nucleic acid inside the cell is also obtained in addition to the number and size of cells by using information of scattered light and fluorescence obtained by applying light from a light source such as a laser to a cell stained with a fluorescent dye or the like flowing through the cell. The flow cytometry method has been adopted in a hemocyte counter from a fully automatic measurement of reticulocytes (RET), and a nucleic acid in the reticulocytes is stained with a nucleic acid-staining fluorescent dye to detect a difference from mature erythrocytes by fluorescence intensity (Non-Patent Literature 1). Here, the reticulocytes are immature erythrocytes after enucleation in which RNA remains and become mature erythrocytes after 24 to 48 hours, and RNA gradually disappears accordingly. Therefore, measurement of the number (and ratio) of reticulocytes is important for assessing erythrocyte hematopoietic ability in a bone marrow. A degree of maturity of reticulocytes is divided into three fractions in proportion to an RNA content, and these fractions are collectively evaluated as an immature reticulocyte fraction (IRF). When a ratio of IRF is compared to the number of reticulocytes, it is clinically useful for differentiating hemolytic anemia, aplastic anemia, or the like and as an index of hematopoietic state after chemotherapy for cancer (Non-Patent Literature 2).

In addition, there is an immature platelet fraction (IPF) as an analysis parameter in which the detection principle of reticulocytes is applied. Here, attempts to detect immature platelets that have just been released from a bone marrow have been made for a long time, and there are many reports under the name of reticulated platelets (RP). RP is said to contain many RNAs in a cytoplasm compared to mature platelets and has been initially reported as platelets stained with new methylene blue dye observed under a microscope. After that, a method has been developed to measure RP stained with a nucleic acid staining dye with a flow cytometer, and it has been shown that RP has increased during idiopathic thrombocytopenic purpura (ITP) and bone marrow recovery after chemotherapy or hematopoietic stem cell transplantation. RP in peripheral blood is thought to reflect a platelet-producing ability of bone marrow, and being able to estimate the platelet-producing ability of bone marrow without performing bone marrow aspiration is useful in situations where a bone marrow examination is difficult, for example. For the measurement of RP, an IPF analysis system, in which the principle of a reticulocyte measurement function installed in a general blood analyzer is applied, is clinically used. The degree of maturity of this immature platelet fraction (IPF) is also divided into three fractions, and it is evaluated that immaturity is higher in descending order of fluorescence intensity in the divided regions.

Conventionally, many techniques for measuring the immature reticulocyte fraction (IRF) and the immature platelet fraction (IPF) using a flow cytometry type automatic blood cell analyzer are known. However, all clinically used devices emit a single color of fluorescence as a fluorescent dye. Therefore, nucleic acid information to be obtained is limited to information obtained from one fluorescence wavelength band. Thus, the nucleic acid information to be obtained includes not only information derived from RNA but also information derived from DNA, and it is not possible to extract only RNA information from the information. As a result, it is considered that measured values of IRF and IPF are obtained as values larger than the true values, and there is a problem that it is not possible to measure the true immaturity of reticulocytes and reticulated platelets.

DNA information obtained as a result of analysis of a blood sample is thought to become a potential indicator showing abnormalities in blood cells (for example, the presence of various bodies (such as Howell-Jolly body and Pappenheimer body), malaria parasite, Babesia, Theileria, Trypanosoma, and microphilia of filaria, and the like). However, since the nucleic acid information obtained from a conventional automatic blood cell analyzer has to be a mixture of RNA information and DNA information, it has not been possible to cause potentially useful DNA information to become manifest.

By the way, there has been proposed a technique for analyzing a blood sample stained with acridine orange (AO), which is a fluorescent dye (metachromatic orthochromatic dye) showing a modulation phenomenon (metachromasia) in which a tissue component exhibits a dyeability different from an original color tone of the dye, by a flow cytometry type automatic blood cell analyzer (Patent Literature 1). Specifically, Patent Literature 1 discloses a technique for normalizing the fluorescence intensities of blood cells stained with AO by the sizes and shapes of the cells according to each scattered light, based on the result of the flow cytometry to obtain a fluorescence concentration of each cell and classifying the cells in the blood sample based on the fluorescence concentration of each cell thus obtained. Then, according to the technique described in Patent Literature 1, it is said that a parameter that is an indicator of immaturity can be calculated from the reticulocytes classified by the above method, depending on the amount of RNA in the reticulocytes. In addition, it is said that the number of reticulated platelets that can be provided as an indicator of immaturity depending on the amount of RNA in platelets can be measured from the platelets similarly classified.

### Citation List

### Patent Literature

PTL 1: Patent Literature 1: US2009/0130647A1

### Non-Patent Literature

NPL 1: Non-Patent Literature 1: Yasunori Abe et al., Journal of Thrombosis and Hemostasis, The Japanese Society on Thrombosis and Hemostasis, 18 (4): 289-301 (2007)
NPL 2: Non-Patent Literature 2: Takayuki Takubo, The Journal of the Japanese Society of Internal Medicine, The Japanese Society of Internal Medicine, Vol. 100, No. 11: 3230-3239 (2011)

### SUMMARY OF THE INVENTION

### Technical Problem

However, the technique described in Patent Literature 1 simply classifies (clusters) the cells in the blood sample based on the fluorescence concentration of each cell normalized by the sizes and shapes of particles in the blood sample. Thus, sufficient clinically useful information has not yet been obtained.

Thus, an object of the present invention is to provide a means capable of obtaining more clinically useful information when particles contained in a blood sample are analyzed using a metachromatic orthochromatic dye.

### Solution to Problem

The present inventor has made diligent studies in view of the above problem. As a result, the present inventor has found that the above problem can be solved by classifying (clustering) particles in a blood sample into a plurality of particle clusters based on a fluorescence concentration normalized by the size of each particle contained in the blood sample and then creating an RNA amount histogram in which a first fluorescence intensity is a class and a DNA amount histogram in which a second fluorescence intensity is a class for at least one particle cluster included in the plurality of particle clusters, and the present invention is completed accordingly.

That is, according to one aspect of the present invention, there is provided a particle analysis method for analyzing particles contained in a blood sample according to appended claim 1.

According to another aspect of the present invention, a particle analyser according to appended claim 10 is provided.

### Brief Description of Drawings

[fig.1]FIG. 1 is a schematic diagram showing preparation of a measurement sample.
[fig.2]FIG. 2 is a diagram showing a system configuration of an apparatus for carrying out a particle analysis method according to an aspect of the present invention.
[fig.3]FIG. 3 is a system diagram showing an outline of a flow cytometer as an embodiment of the apparatus for carrying out the particle analysis method according to an aspect of the present invention.
[fig.4]FIG. 4 is a measurement example of a two-dimensional scattergram (FS × SS cytogram) of forward-scattered light (FS) and lateral-scattered light (SS).
[fig.5]FIG. 5 is a measurement example of a two-dimensional scattergram (FL1 × FL2 cytogram) of a first fluorescence (FL1) and a second fluorescence (FL2).
[fig.6]FIG. 6 is a two-dimensional plot diagram (also referred to as "RNP diagram" in the present specification) of a fluorescence concentration (CRc) of a first fluorescence (FL1) and a fluorescence concentration (CDc) of a second fluorescence (FL2) created based on a result obtained by determining the CRc and the CDc in each particle by processing of "normalization".
[fig.7A]FIG. 7A is an RNA amount histogram and a DNA amount histogram created by separating several particle clusters from the RNP diagram shown in FIG. 6 by gating. Specifically, FIG. 7A is the RNA amount histogram (left in FIG. 7A) and the DNA amount histogram (right in FIG. 7A) for an erythrocyte cluster (RBCn).
[fig.7B]FIG. 7B is an RNA amount histogram and a DNA amount histogram created by separating several particle clusters from the RNP diagram shown in FIG. 6 by gating. Specifically, FIG. 7B is the RNA amount histogram (left in FIG. 7B) and the DNA amount histogram (right in FIG. 7B) for a platelet cluster (PLTn).
[fig.7C]FIG. 7C is an RNA amount histogram and a DNA amount histogram created by separating several particle clusters from the RNP diagram shown in FIG. 6 by gating. Specifically, FIG. 7C is the RNA amount histogram (left in FIG. 7C) and the DNA amount histogram (right in FIG. 7C) for the nucleated cell cluster (NCn).
[fig.8A]FIG. 8A is an example of a two-dimensional plot diagram created for the particle cluster (erythrocyte cluster (RBCn) in this case), for which the RNA amount histogram and the DNA amount histogram are created. In the two-dimensional plot diagram, intensity of the first fluorescence (FL1) or intensity of the second fluorescence (FL2) of each particle included in the particle cluster is one axis and the size of each particle included in the particle cluster is the other axis.
[fig.8B]FIG. 8B is an example of a two-dimensional plot diagram created for the particle cluster (platelet cluster (PLTn) in this case), for which the RNA amount histogram and the DNA amount histogram are created. In the two-dimensional plot diagram, intensity of the first fluorescence (FL1) or intensity of the second fluorescence (FL2) of each particle included in the particle cluster is one axis and the size of each particle included in the particle cluster is the other axis.
[fig.8C]FIG. 8C is an example of a two-dimensional plot diagram created for the particle cluster (nucleated cell cluster (NCn) in this case), for which the RNA amount histogram and the DNA amount histogram are created. In the two-dimensional plot diagram, intensity of the first fluorescence (FL1) or intensity of the second fluorescence (FL2) of each particle included in the particle cluster is one axis and the size of each particle included in the particle cluster is the other axis.
[fig.9A]FIG. 9A is an RNP diagram created when a reticulocyte fraction is measured by applying the present invention to a blood sample without morphological findings in an Example described later.
[fig.9B]FIG. 9B shows the RNA amount histogram (left in FIG. 9B) and the DNA amount histogram (right in FIG. 9B) created when the reticulocyte fraction is measured by applying the present invention to the blood sample without morphological findings in an Example described later.
[fig.9C]FIG. 9C is a two-dimensional plot diagram created when the reticulocyte fraction is measured by applying the present invention to the blood sample without morphological findings in an Example described later, in which the intensity of the first fluorescence (FL1) of each particle included in the erythrocyte cluster or the platelet cluster is a horizontal axis and the size of each particle included in the cluster is a vertical axis.
[fig.9D]FIG. 9D is a two-dimensional plot diagram created when the reticulocyte fraction is measured by applying the present invention to the blood sample without morphological findings in an Example described later, in which the intensity of the second fluorescence (FL2) of each particle included in the erythrocyte cluster or the platelet cluster is the horizontal axis and the size of each particle included in the cluster is the vertical axis.
[fig.10A]FIG. 10A is an RNP diagram created when a reticulocyte fraction is measured by applying the present invention to the blood sample with morphological findings of erythrocyte in an Example described later.
[fig.10B]FIG. 10B shows the RNA amount histogram (left in FIG. 10B) and the DNA amount histogram (right in FIG. 10B) created when the reticulocyte fraction is measured by applying the present invention to the blood sample with morphological findings of erythrocyte in an Example described later.
[fig.10C]FIG. 10C is a two-dimensional plot diagram created when the reticulocyte fraction is measured by applying the present invention to the blood sample with morphological findings of erythrocyte in an Example described later, in which the intensity of the first fluorescence (FL1) of each particle included in the erythrocyte cluster or the platelet cluster is the horizontal axis and the size of each particle included in the cluster is the vertical axis.
[fig.10D]FIG. 10D is a two-dimensional plot diagram created when the reticulocyte fraction is measured by applying the present invention to the blood sample with morphological findings of erythrocyte in an Example described later, in which the intensity of the second fluorescence (FL2) of each particle included in the erythrocyte cluster or the platelet cluster is the horizontal axis and the size of each particle included in the cluster is the vertical axis.
[fig.11A]FIG. 11A is an RNP diagram created when measurement is performed by applying the second aspect of the present invention to the blood sample without morphological findings of erythrocyte in an Example described later.
[fig.11B]FIG. 11B is a fluorescence intensity ratio histogram that is created when measurement is performed by applying the second aspect of the present invention to the blood sample without morphological findings of erythrocyte in an Example described later and in which a fluorescence intensity ratio (FL1/FL2), which is a value of a ratio of the intensity of the first fluorescence (FL1) to the intensity of the second fluorescence (FL2) of each particle included in an erythrocyte cluster is a class.
[fig.11C]FIG. 11C is an enlarged view of a region near a threshold value (FL1/FL2 = 2.0) in FIG. 11B.
[fig.12A]FIG. 12A is an RNP diagram created when measurement is performed by applying the second aspect of the present invention to the blood sample with morphological findings (HJ body) of erythrocyte in an Example described later.
[fig.12B]FIG. 12B is a fluorescence intensity ratio histogram that is created when measurement is performed by applying the second aspect of the present invention to the blood sample with morphological findings (HJ body) of erythrocyte in an Example described later and in which the fluorescence intensity ratio (FL1/FL2), which is the value of the ratio of the intensity of the first fluorescence (FL1) to the intensity of the second fluorescence (FL2) of each particle included in the erythrocyte cluster is a class.
[fig.12C]FIG. 12C is an enlarged view of the region near the threshold value (FL1/FL2 = 2.0) in FIG. 12B.
[fig.13A]FIG. 13A is an RNP diagram created when measurement is performed by applying the second aspect of the present invention to the blood sample with morphological findings (nucleated erythrocyte (NRBC)) of erythrocyte in an Example described later.
[fig.13B]FIG. 13B is a fluorescence intensity ratio histogram that is created when measurement is performed by applying the second aspect of the present invention to the blood sample with morphological findings (nucleated erythrocyte (NRBC)) of erythrocyte in an Example described later and in which the fluorescence intensity ratio (FL1/FL2), which is the value of the ratio of the intensity of the first fluorescence (FL1) to the intensity of the second fluorescence (FL2) of each particle included in the erythrocyte cluster is a class.
[fig.13C]FIG. 13C is an enlarged view of the region near the threshold value (FL1/FL2 = 2.0) in FIG. 12B.

### DETAILED DESCRIPTION

Hereinafter, embodiments of the present invention will be described with reference to the accompanying drawings.

An aspect (first aspect) of the present invention is a particle analysis method of analyzing particles contained in a blood sample, including staining the particles with a metachromatic orthochromatic dye, irradiating the stained particles with light, measuring intensity of a first fluorescence derived from a stacking component of the metachromatic orthochromatic dye and intensity of a second fluorescence derived from an intercalation component of the metachromatic orthochromatic dye, the first fluorescence and the second fluorescence being emitted by each particle contained in the blood sample, normalizing the intensity of the first fluorescence and the intensity of the second fluorescence, emitted by each of the particles, by the size of each of the particles to obtain a fluorescence concentration of each of the first fluorescence and the second fluorescence in each of the particles, clustering each of the particles into a plurality of particle clusters including at least two of an erythrocyte cluster, a platelet cluster and a nucleated cell cluster, in a two-dimensional plot of the fluorescence concentration obtained by the normalization, and creating an RNA amount histogram in which the intensity of the first fluorescence is a class and a DNA amount histogram in which the intensity of the second fluorescence is a class for at least one particle cluster included in the plurality of particle clusters.

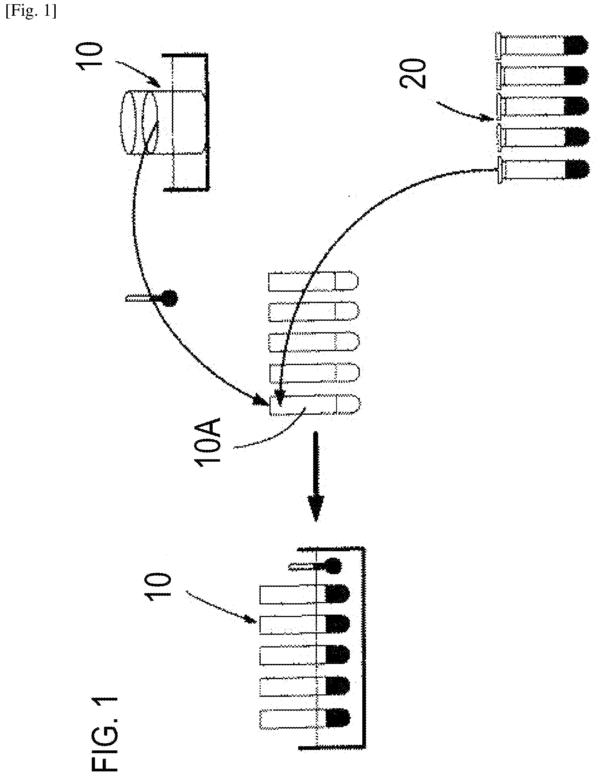

FIG. 1 is a schematic diagram showing preparation of a measurement sample. In the particle analysis method according to the present invention, first, a sample (blood sample) containing particles in blood is provided, and a predetermined orthochromatic dye (metachromatic orthochromatic dye) is used (usually, the dye and the blood sample are mixed) to prepare the measurement sample. As a result, the particles contained in the blood sample are stained with the predetermined orthochromatic dye. In the analysis of particles by the flow cytometry method, the measurement sample prepared above is irradiated with light, whereby scattered light and a fluorescence generated from the particles contained in the sample are detected as electrical signals. Then, based on the detected electrical signal, the particles contained in the sample are analyzed.

### (Preparation of measurement sample)

In the present aspect, as described above, a sample (blood sample) containing particles in blood is provided as a sample to be measured (measurement sample), and a predetermined orthochromatic dye (metachromatic orthochromatic dye) is used (usually, the dye and the blood sample are mixed) to prepare the measurement sample. In this case, for example, as shown in FIG. 1, when a required amount of orthochromatic dye 10 is dispensed in a fixed amount, the orthochromatic dye is warmed in a range of 20 to 50°C. To an orthochromatic dye 10A thus warmed and dispensed, a sample (blood sample) 20 containing particles in blood is added, and the mixture is stirred for 5 to 10 seconds. A measurement sample 30 thus obtained is kept warm in the range of 20 to 50°C and held for 10 to 40 seconds. As a result, in the present invention, the preparation of the measurement sample 30 can be completed in 15 to 60 seconds.

In this case, for example, 1 mL each of the metachromatic orthochromatic dye 10 is dispensed, and to the dispensed metachromatic orthochromatic dye 10A, 2 µL each of the blood sample 20 prepared so that the number of particles to be measured is about 1 × 10⁷ particles/µL is added. As the metachromatic orthochromatic dye 10, for example, 0.5 to 1.5 mg/dL of acridine orange prepared using a tris buffer solution having a pH of 7.4 can be used. In particular, a dye concentration is preferably about 0.75 mg/dL. This metachromatic orthochromatic dye 10 is dispensed into 1 mL and warmed to 45°C during the dispensing, 2 µL of the blood sample 20 is added to 1 mL of the orthochromatic dye 10A warmed, and the mixture is stirred for 5 seconds. The temperature of the obtained sample is kept at 45°C and held for 30 seconds, whereby the measurement sample 30 as a proper sample can be prepared. Alternatively, a measurement sample may be prepared by sequentially adding a buffer solution, such as a phosphate buffer solution or a tris buffer solution having a pH of 6.4 to pH 8.2, and the blood sample 20 to separately freeze-dried acridine orange.

"Metachromasia" is a term originally meant for a modulation phenomenon in which a component stained with a dye exhibits a dyeability different from an original color tone of the dye. In the present specification, this term is used to define a "metachromatic orthochromatic dye" as a dye having a property that emits a plurality of fluorescences having different wavelengths depending on the type of target to be stained with the metachromatic orthochromatic dye or a dyeing method. Specific examples of the metachromatic orthochromatic dye include acridine orange (AO), proflavine, acriflavine, atebrin, and the like. These metachromatic orthochromatic dyes can be used without particular limitation as long as they are dyes in which the wavelengths of fluorescences emitted by a stacking component and an intercalation component, which will be described later, are different. However, the fluorescence emitted by the stacking component of the metachromatic orthochromatic dye is preferably an orange fluorescence, and the fluorescence emitted by the intercalation component is preferably a green fluorescence. From this point of view, acridine orange (AO) is particularly preferably used as the metachromatic orthochromatic dye.

### (Measurement and analysis of prepared sample)

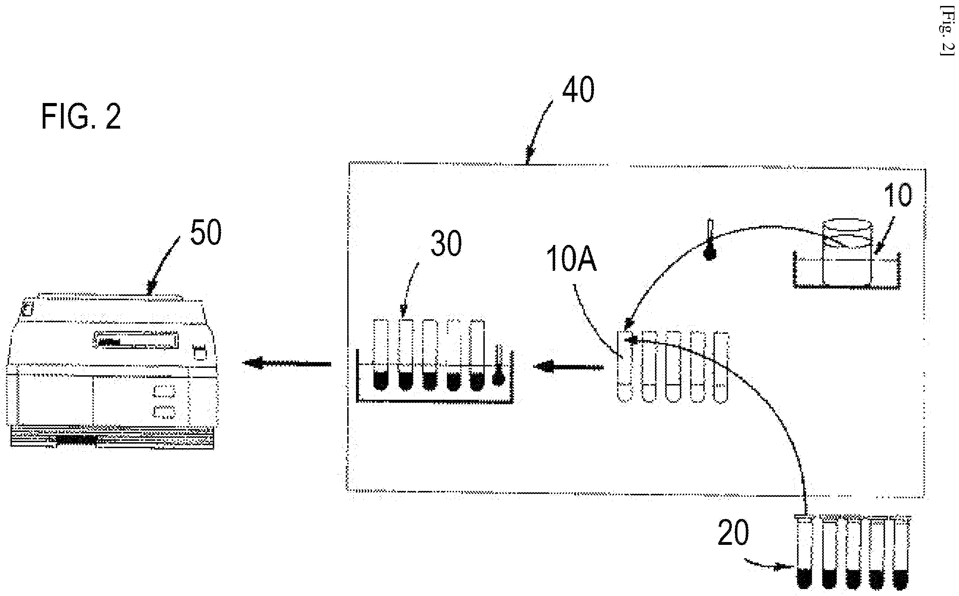

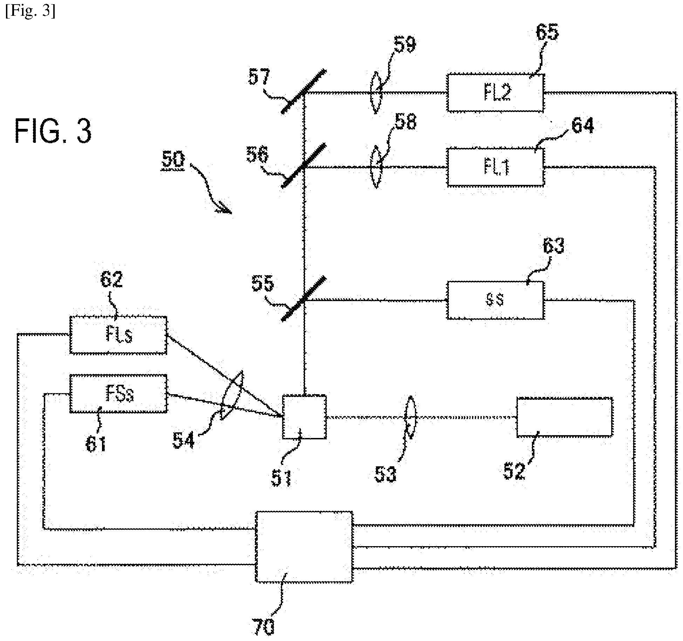

FIG. 2 is a diagram showing a system configuration of an apparatus for carrying out the particle analysis method according to an aspect of the present invention. Fig. 3 is a system diagram showing an outline of a flow cytometer as an embodiment of the apparatus for carrying out the particle analysis method according to the present aspect.

As shown in FIG. 2 and FIG. 3, the apparatus is constituted of a sample preparation unit 40 that prepares the measurement sample 30 described above with reference to FIG. 1 and a flow cytometer 50 that analyzes the measurement sample 30 by the flow cytometry method. The flow cytometer 50 has a flow cell 51 as a detection region through which the measurement sample 30 flows and a laser light source 52 that is a light source that irradiates the measurement sample 30 (specifically, particles contained in the sample) flowing through the flow cell 51 with light. The laser light source 52 is disposed with respect to the flow cell 51 through an irradiation-light condensing lens 53. In the flow cytometer 50, a detector for small-angled forward-scattered light (FSs) 61 and a detector for large-angled forward-scattered light (FLs) 62 that detect forward-scattered light generated from each particle in the measurement sample 30 by light irradiation of the measurement sample 30 in the flow cell 51 are arranged through a scattered-light condensing lens 54. The arrangement of the detector for large-angled forward-scattered light (FLs) 62 is not essential. Further, in the flow cytometer 50, a lateral-scattered light detector (SS) 63 that detects scattered light in the lateral direction generated from each particle in the measurement sample 30 by light irradiation of the measurement sample 30 in the flow cell 51 is disposed through a beam splitter 55. Furthermore, in the flow cytometer 50, a first fluorescence detector (FL1) 64 and a second fluorescence detector (FL2) 65 for detecting the respective two fluorescences having different wavelengths and generated from each particle in the measurement sample 30 by light irradiation of the measurement sample 30 in the flow cell 51 are arranged through beam splitters 56 and 57 and wavelength-selective filters 58 and 59, respectively. A dichroic mirror may be used instead of the beam splitter. Each of the above-mentioned detectors 61, 62, 63, 64, and 65 functions as a light detector (scattered-light detector, fluorescence detector) that detects intensities of the scattered light and fluorescence generated from each particle contained in the measurement sample 30 irradiated with light.

The flow cytometer 50 has a processor (CPU) 70. This processor (CPU) 70 also functions as a data processing part that analyzes particles contained in a blood sample based on the intensity of scattered light and fluorescence generated from each particle detected by the light detector and carries out a process related to data processing (calculation of fluorescence concentration by normalization of fluorescence intensity, clustering of particles contained in a sample, and creation of a nucleic acid amount histogram) in the particle analysis method according to the present aspect.

Subsequently, the particle analysis method according to the present invention using the apparatus having the configurations shown in FIG. 2 and FIG. 3 will be described in detail.

First, the measurement sample 30 prepared by the sample preparation unit 40 described above is supplied to the flow cell 51 of the flow cytometer 50 to start the analysis. When the measurement sample 30 is supplied to the flow cell 51, the laser light source 52 irradiates the measurement sample 30 (specifically, the particles contained in the sample) flowing through the flow cell 51 with light. Here, the wavelength of the irradiation light is not particularly limited, but a central wavelength of the irradiation light is preferably 408 nm, 445 nm, 473 nm or 488 nm.

When the measurement sample 30 is irradiated with light, forward-scattered light (forward-scattered light (FS)) is generated from each particle contained in the measurement sample 30, and the forward-scattered light (FS) is detected by the detector for small-angled forward-scattered light (FSs) 61 and the detector for large-angled forward-scattered light (FLs) 62. When the measurement sample 30 is irradiated with light, scattered light in the lateral direction (lateral-scattered light (SS)) is generated from each particle contained in the measurement sample 30, and the lateral-scattered light (SS) is detected by the lateral-scattered light detector (SS) 63. In addition, when the measurement sample 30 is irradiated with light, fluorescence is generated from each particle contained in the measurement sample 30. Here, in the method according to the present aspect, the particles contained in the blood sample are stained with the metachromatic orthochromatic dye. Therefore, when the measurement sample 30 is irradiated with light, a plurality of (for example, two) fluorescences having different wavelengths from each other are generated from each particle contained in the measurement sample 30. Specifically, the plurality of fluorescences include a fluorescence (also referred to as "first fluorescence (FL1)" in the present specification) derived from the stacking component of the metachromatic orthochromatic dye and a fluorescence (also referred to as "second fluorescence (FL2)" in the present specification) derived from the intercalation component of the metachromatic orthochromatic dye. The fluorescence (first fluorescence (FL1)) derived from the stacking component is the fluorescence generated by stacking the metachromatic orthochromatic dye to nucleic acid by electrostatic interaction, and is the fluorescence having a central wavelength of about 645 to 655 nm when acridine orange (AO) is used as the dye. The fluorescence intensity of the first fluorescence (FL1) is mainly correlated with an abundance of ribonucleic acid (RNA) among the nucleic acids. On the other hand, the fluorescence (second fluorescence (FL2)) derived from the intercalation component is the fluorescence generated by intercalating the metachromatic orthochromatic dye to nucleic acid, and is the fluorescence having a central wavelength of about 520 to 530 nm when acridine orange (AO) is used as the dye. The fluorescence intensity of the second fluorescence (FL2) is mainly correlated with an abundance of deoxyribonucleic acid (DNA) among the nucleic acids. The first fluorescence (FL1) and the second fluorescence (FL2) generated from each particle contained in the measurement sample 30 by light irradiation of the measurement sample 30 are detected by the first fluorescence detector (FL1) 64 and the second fluorescence detector (FL2) 65, respectively.

As described above, the intensity of the scattered light (forward-scattered light (FS) and lateral-scattered light (SS)) and the intensity of the fluorescence (first fluorescence (FL1) and second fluorescence (FL2)) detected by the detectors are each converted into an electrical signal at the detector and transmitted to the processor (CPU) 70. Then, the processor (CPU) 70 performs various data processing using the electrical signal thus obtained. For example, the processor (CPU) 70 calculates a parameter related to the size of each particle based on the intensity of the forward-scattered light (FS), and calculates a parameter related to the size of each particle and an amount of granules contained in each particle based on the intensity of the lateral-scattered light (SS). The processor (CPU) 70 calculates parameters related to an amount of the stacking component and an amount of the intercalation component in each particle, respectively, based on the intensity of the first fluorescence (FL1) and the intensity of the second fluorescence (FL2). Here, as described above, the fluorescence intensity of the first fluorescence (FL1) is mainly correlated with the abundance of ribonucleic acid (RNA) among the nucleic acids, and the fluorescence intensity of the second fluorescence (FL2) is mainly correlated with the abundance of deoxyribonucleic acid (DNA) among the nucleic acids. Therefore, the parameters related to the amount of the stacking component and the amount of the intercalation component in each particle, calculated from the electrical signals derived from the intensity of the first fluorescence (FL1) and the intensity of the second fluorescence (FL2), can be regarded as parameters related to an amount of RNA and an amount of DNA in each particle, respectively.

In the present embodiment, the processor (CPU) 70 then normalizes the intensity of the first fluorescence (FL1) and the intensity of the second fluorescence (FL2) emitted by each particle by the size of each particle. Consequently, the respective fluorescence concentrations of the first fluorescence (FL1) and the second fluorescence (FL2) in each particle can be obtained. According to scattering theory, it is known that the intensity (scattering cross section) of the forward-scattered light (FS) is proportional to the size (diameter) of the particles that emit the forward-scattered light. Therefore, when the intensity of the fluorescence (FL1, FL2) emitted by each particle is divided by the parameter related to the size of each particle (the intensity of the forward-scattered light (FS) or diameter calculated based on this intensity), the fluorescence intensity when it is assumed that the particles have the same size (that is, the fluorescence concentration) can be obtained. In the present specification, this processing is referred to as "normalization". In the present specification, for convenience, the fluorescence concentration of the first fluorescence (FL1) emitted by each particle is referred to as CRc (Cell RNA concentration; intracellular RNA concentration), and the fluorescence concentration of the second fluorescence (FL2) emitted by each particle is referred to as CDc (Cell DNA concentration; intracellular DNA concentration). In the following, some information obtained up to the time of completion of normalization by measuring a blood sample will be described first.

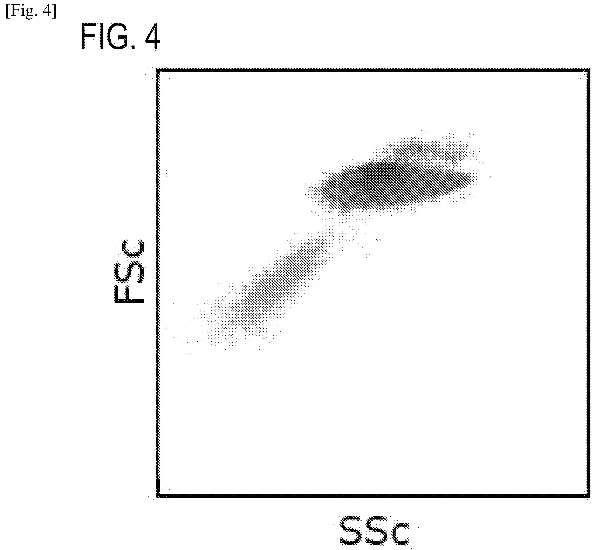

FIG. 4 is a measurement example of a two-dimensional scattergram (FS × SS cytogram) of the forward-scattered light (FS) and the lateral-scattered light (SS). In FIG. 4, a purple event indicates an erythrocyte component, a green event indicates a platelet component, and a blue event indicates a nucleated cell component. As shown in FIG. 4, in the FS × SS cytogram, a cluster of the purple events (erythrocyte components) and a cluster of the blue events (nucleated cell components) are displayed overlapping. Some of the purple events (erythrocyte components) are present in a cluster of the green events (platelet components). Therefore, when the FS × SS cytogram is used as it is, no specific blood cell component can be separated from other blood cell components no matter how gating is applied.

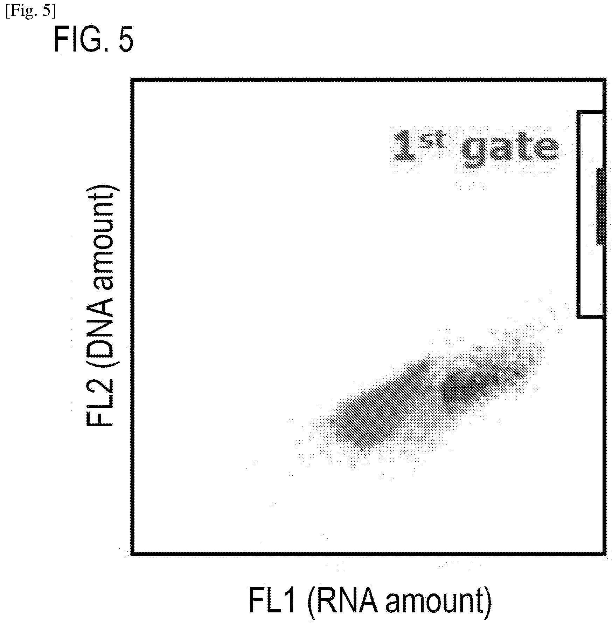

FIG. 5 is a measurement example of a two-dimensional scattergram (FL1 × FL2 cytogram) of the first fluorescence (FL1) and the second fluorescence (FL2). As shown in FIG. 5, also in the FL1 × FL2 cytogram, the cluster of the purple events (erythrocyte components) and the cluster of the green events (platelet components) are displayed overlapping. On the other hand, the cluster of the blue events (nucleated cell components) is present independently in the upper right of the cytogram. Therefore, it is possible to gate only the nucleated cell component by setting a gate for the cluster of the blue events (nucleated cell component) as shown in FIG. 5.

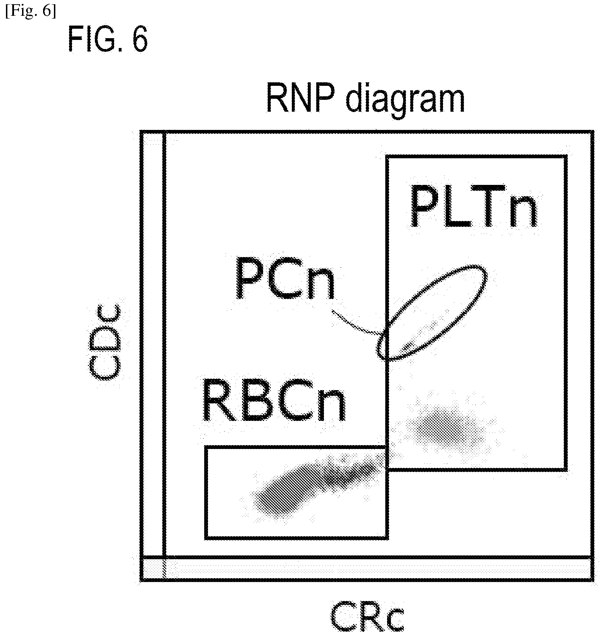

Subsequently, FIG. 6 is a two-dimensional plot diagram (also referred to as "RNP diagram" in the present specification) of the fluorescence concentration (CRc) of the first fluorescence (FL1) and the fluorescence concentration (CDc) of the second fluorescence (FL2) created based on the result obtained by determining the CRc and the CDc in each particle by the processing of "normalization" described above. In the RNP diagram shown in FIG. 6, the blue event (nucleated cell component) that can be deleted by the above-mentioned gating is also displayed for confirming the position of presence. That is, based on the above-mentioned definition, in the RNP diagram, the horizontal axis shows the fluorescence concentration (CRc) of the first fluorescence (FL1) of each particle, and the vertical axis shows the fluorescence concentration (CDc) of the second fluorescence (FL2) of each particle. Reflecting this, it can be seen that the nucleated cell components (nucleated cell cluster; PCn) in which both the RNA concentration and the DNA concentration in the particles are relatively high form a cluster in an upper right region of the RNP diagram. In addition, it can also be seen that the erythrocyte components (erythrocyte cluster; RBCn) in which both the RNA concentration and the DNA concentration in the particles are relatively low form a cluster in a lower left region of the RNP diagram, and the platelet components (platelet cluster; PLTn) in which while the RNA concentration in the particles is relatively high, the DNA concentration is relatively low, form a cluster in a lower right region of the RNP diagram.

As described above, the processing of "normalization" described above is performed to obtain the respective fluorescence concentrations of the first fluorescence (FL1) and the second fluorescence (FL2) in each particle, and thus to create a two-dimensional plot diagram in which these fluorescence concentrations are two axes, whereby the particles contained in the blood sample can be clustered. In the particle analysis method according to the present aspect, it is essential to cluster each particle contained in the blood sample into a plurality of particle clusters including at least two of the erythrocyte cluster, the platelet cluster and the nucleated cell cluster, and it is preferable to cluster each particle contained in the blood sample into a plurality of particle clusters including all of the erythrocyte cluster (RBCn), the platelet cluster (PLTn) and the nucleated cell cluster (NCn).

Next, in the particle analysis method according to the present aspect, from the RNP diagram (FIG. 6) created above, for at least one particle cluster included in the plurality of particle clusters generated by clustering using the RNP diagram, a histogram (also referred to as "RNA amount histogram" in the present specification) in which the intensity (CRc) of the first fluorescence (FL1) is a class and a histogram (also referred to as "DNA amount histogram" in the present specification) in which the intensity (CDc) of the second fluorescence (FL2) is a class are created.

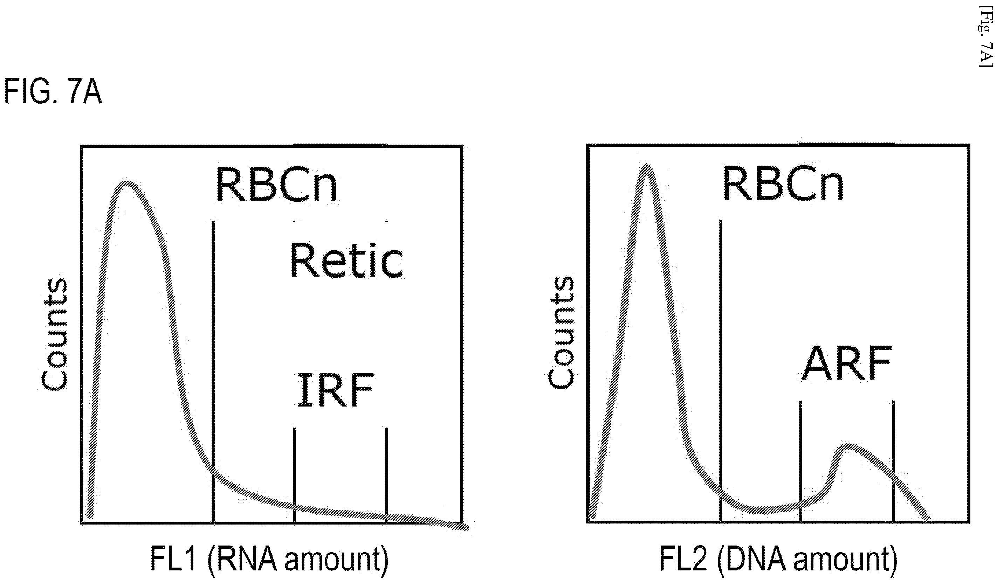

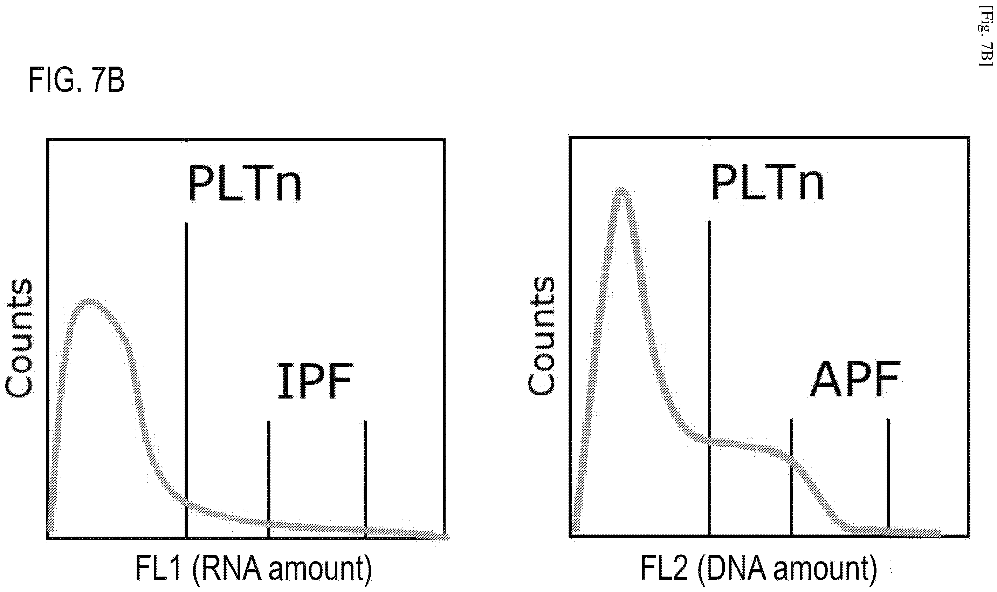

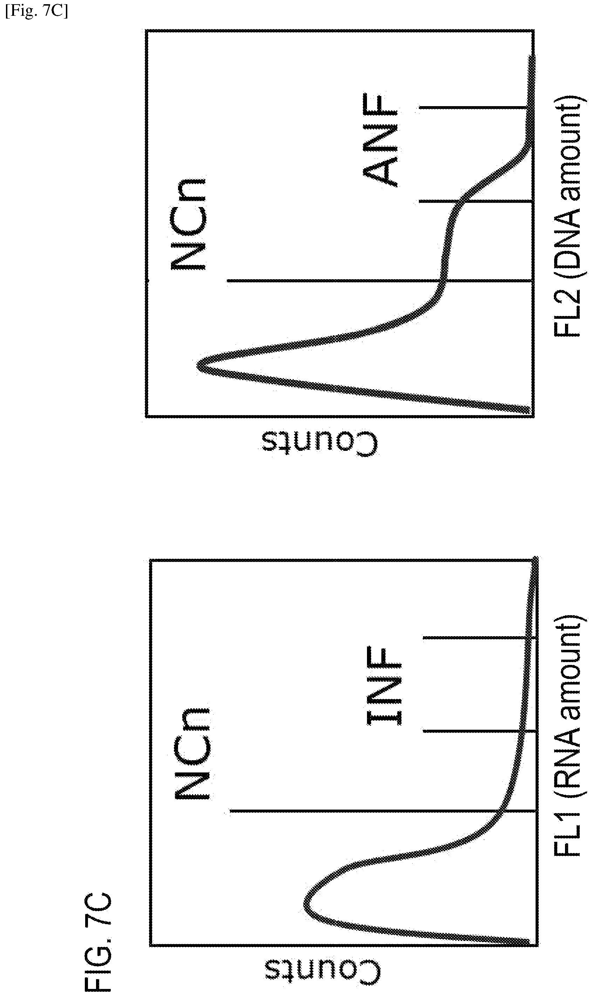

FIG. 7A is an RNA amount histogram (left in FIG. 7A) and a DNA amount histogram (right in FIG. 7A) for the erythrocyte cluster (RBCn), which are created by separating the erythrocyte cluster (RBCn) from the RNP diagram shown in FIG. 6 by gating. Similarly, FIG. 7B is an RNA amount histogram (left in FIG. 7B) and a DNA amount histogram (right in FIG. 7B) for the platelet cluster (PLTn), which are created by separating the platelet cluster (PLTn) from the RNP diagram shown in FIG. 6 by gating. FIG. 7C is an RNA amount histogram (left in FIG. 7C) and a DNA amount histogram (right in FIG. 7C) for the nucleated cell cluster (NCn), which are created by separating the nucleated cell cluster (NCn) from the RNP diagram shown in FIG. 6 by gating.

The particle analysis method according to the present aspect preferably further includes analyzing the particles contained in the blood sample based on the RNA amount histogram and the DNA amount histogram for at least one particle cluster created above. This analysis may be performed by the processor (CPU) 70 (data processing part) included in the flow cytometer 50, may be performed by another computer, or may be performed by a medical professional such as a doctor, a nurse, or a clinical laboratory technician.

Specifically, for example, based on the RNA amount histogram (left in FIG. 7A) for the erythrocyte cluster (RBCn) shown in FIG. 7A, the number or ratio of reticulocytes (Retic) contained in the blood sample and/or an immature reticulocyte fraction (IRF) of the blood sample can be measured. Here, the RNA amount histogram (left in FIG. 7A) for the erythrocyte cluster (RBCn) shown in FIG. 7A is created using data of the erythrocyte cluster separated through gating using the RNP diagram. Therefore, data of the FL1 (RNA amount) in each plot in the RNA amount histogram reflects the amount of the stacking component in each particle with high accuracy, and it is extremely unlikely that a false high value including the DNA amount is shown as in the conventional technique. Therefore, the particle analysis method according to the present aspect has an advantage that, based on the RNA amount histogram for the erythrocyte cluster (RBCn) shown in FIG. 7A, various parameters (the number or ratio of reticulocytes contained in a blood sample and/or the immature reticulocyte fraction (IRF) of the blood sample) can be measured with extremely high accuracy. In reticulocytes, the RNA amount in blood cells is larger than that in normal erythrocytes. Therefore, when a predetermined threshold value is set on the horizontal axis in the RNA amount histogram for the erythrocyte cluster (RBCn) shown in FIG. 7A, the particles in the erythrocyte cluster in which the RNA amount is equal to or more than the threshold value can be determined as reticulocytes. Further, a region on the horizontal axis corresponding to reticulocytes is divided into three according to the fluorescence intensity (RNA amount) of FL1, and divided into three regions: HFR (High Fluorescence Ratio), MFR (Middle Fluorescence Ratio), and LFR (Low Fluorescence Ratio) from a side with the highest fluorescence intensity, and the immature reticulocyte fraction (IRF) can be measured as immaturity of the reticulocytes from the number or ratio of the reticulocytes included in each region.

In the particle analysis method according to the present aspect, unlike the conventional technique, for at least one particle cluster, information on the DNA amount of each particle contained in the blood sample can be obtained as the DNA amount histogram by being separated from information on the RNA amount. Therefore, analysis using the DNA amount histogram described above also provides useful findings. For example, according to the particle analysis method according to the present aspect, the presence or absence of an abnormality in the erythrocyte cluster can be determined based on the DNA amount histogram for the erythrocyte cluster (RBCn) shown in FIG. 7A. Here, the "abnormality" is a concept including all states in which the DNA amount in the particles included in the erythrocyte cluster increases as compared with the normal state. Examples of the "abnormality" include the presence of various bodies (such as Howell-Jolly body and Pappenheimer body), malaria parasite, Babesia, Theileria, Trypanosoma, and microphilia of filaria, and the like in the particles included in the erythrocyte cluster. That is, in the particles in the erythrocyte cluster having these "abnormalities", the DNA amount in the blood cells is large as compared with normal erythrocytes. Therefore, when a predetermined threshold value is set on the horizontal axis in the DNA amount histogram for the erythrocyte cluster (RBCn) shown in FIG. 7A, if there is a particle in the erythrocyte cluster in which the DNA amount is equal to or more than the threshold value, it can be determined that the particle is likely to have some of the above "abnormalities" (Abnormal Reticulocyte Fraction; ARF).

Hereinabove, the specific embodiment of the analysis based on the RNA amount histogram or the DNA amount histogram for the erythrocyte cluster (RBCn) has been described with reference to FIG. 7A. However, a similar analysis can be performed based on the RNA amount histogram or the DNA amount histogram for the platelet cluster (PLTn) shown in FIG. 7B, and a similar analysis can be performed based on the RNA amount histogram or the DNA amount histogram for the nucleated cell cluster (NCn) shown in FIG. 7C. For example, there is an advantage that various parameters (Immature Platelet Fraction (IPF) in the blood sample) can be measured with extremely high accuracy based on the RNA amount histogram for the platelet cluster (PLTn) shown in FIG. 7B. When a predetermined threshold value is set on the horizontal axis in the DNA amount histogram for the platelet cluster (PLTn) shown in FIG. 7B, if there is a particle in the platelet cluster in which the DNA amount is equal to or more than the threshold value, it can be determined that the particle is likely to have some of the above "abnormalities" (Abnormal Platelet Fraction; APF). Similarly, based on the RNA amount histogram and the DNA amount histogram for the nucleated cell cluster (NCn), the immaturity (Immature Nucleated cell Fraction) of particles included in the nucleated cell cluster (NCn) and the presence or absence of abnormality (Abnormal Nucleated cell Fraction) can be determined respectively.

In a preferred embodiment of the particle analysis method according to the present aspect, for the particle cluster in which the RNA amount histogram and the DNA amount histogram are created as described above, a two-dimensional plot diagram is created in which the intensity of the first fluorescence (FL1) or the intensity of the second fluorescence (FL2) of each particle included in the particle cluster is one axis and the size of each particle included in the particle cluster is the other axis.

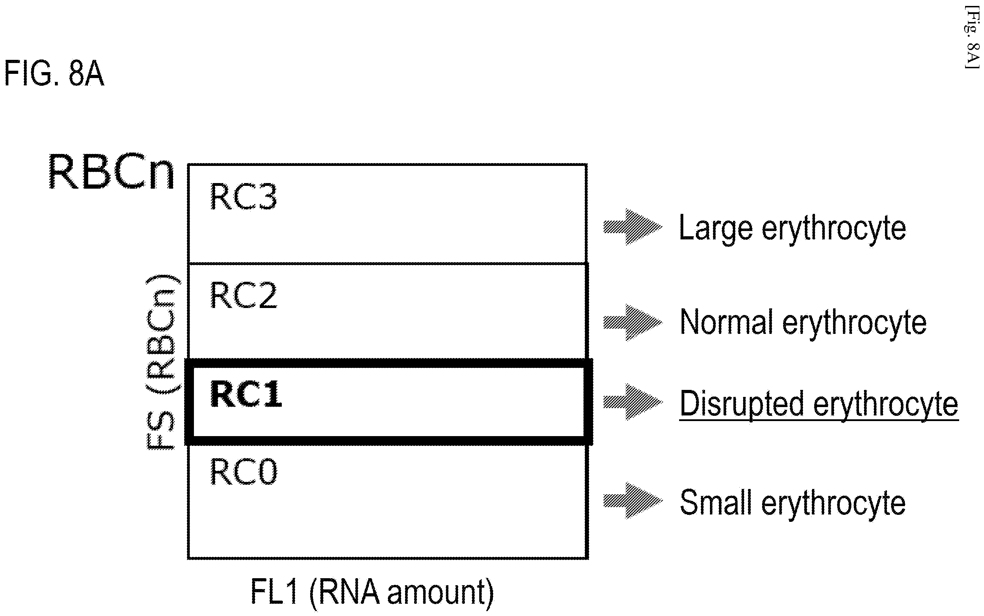

As an example of such a two-dimensional plot diagram, Fig. 8A is a two-dimensional plot diagram in which, for the erythrocyte cluster (RBCn), the intensity of the first fluorescence (FL1) of each particle included in the cluster is the horizontal axis and the size (in this case, the intensity of the forward-scattered light (FS)) of each particle included in the cluster is the vertical axis. Then, in the two-dimensional plot diagram, the vertical axis (intensity of forward-scattered light (FS)) is divided into a plurality of regions (in this case, four regions RC0 to RC3 from the smallest to the largest). Therefore, erythrocyte clusters can be reclassified into a plurality of subclusters based on the number or ratio of particles in each of the four regions. In this case, the erythrocyte cluster is reclassified into four subclusters from the largest particle size: a large erythrocyte subcluster (RC3), a normal erythrocyte subcluster (RC2), a disrupted erythrocyte subcluster (RC1), and a small erythrocyte subcluster (RC0).

Here, since the horizontal axis of the two-dimensional plot diagram shown in FIG. 8A shows the intensity of the first fluorescence (FL1), which is an indicator of the RNA amount (in other words, immaturity) in each particle, information on the immaturity and information on the size of each particle included in the erythrocyte cluster can be simultaneously obtained based on the two-dimensional plot diagram showing the erythrocyte cluster (RBCn) shown in FIG. 8A. As a result, it opens up the possibility of obtaining clinically useful findings that cannot be grasped from only one of the information (for example, the value of the immature reticulocyte fraction (IRF)).

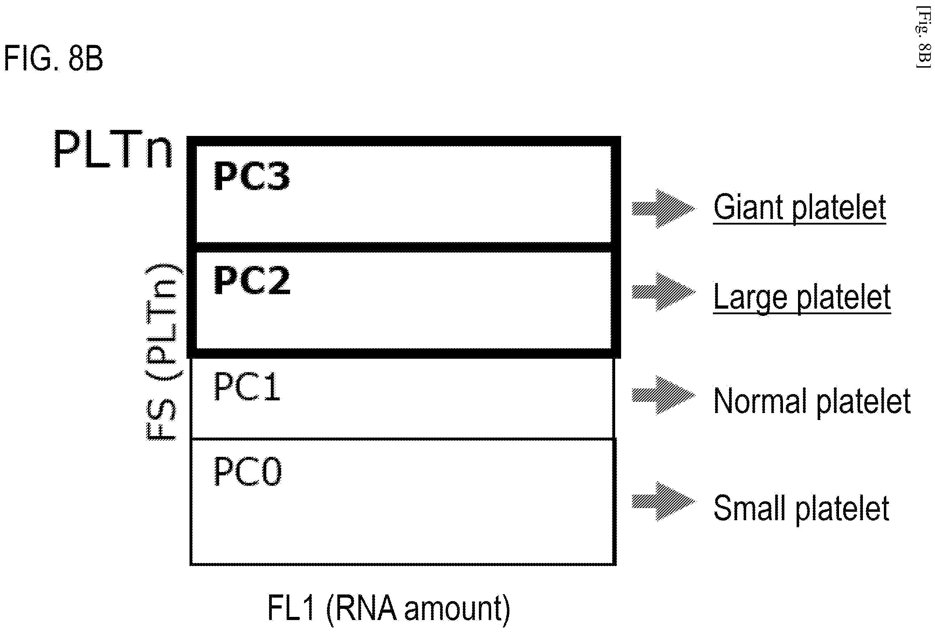

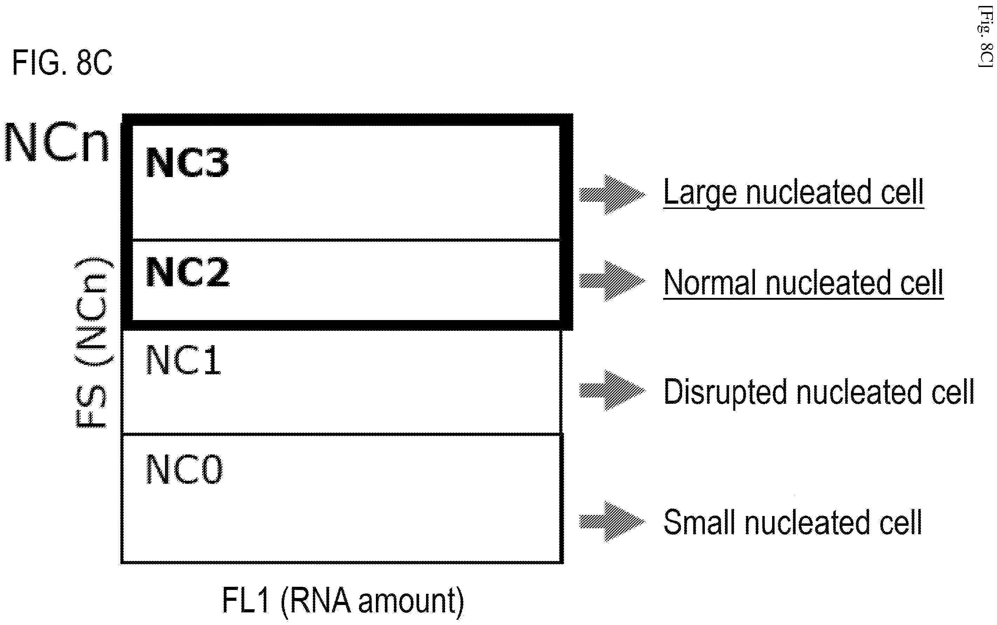

FIG. 8B and FIG. 8C are two-dimensional plot diagrams similarly created for the platelet cluster (PLTn) and the nucleated cell cluster (NCn), respectively. Specifically, FIG. 8B is a two-dimensional plot diagram in which, for the platelet cluster (PLTn), the intensity of the first fluorescence (FL1) of each particle included in the cluster is the horizontal axis and the size (in this case, the intensity of the forward-scattered light (FS)) of each particle included in the cluster is the vertical axis. Then, in the two-dimensional plot diagram, the vertical axis (intensity of forward-scattered light (FS)) is divided into a plurality of regions (in this case, four regions PC0 to PC3 from the smallest to the largest). Therefore, platelet clusters can be reclassified into a plurality of subclusters based on the number or ratio of particles in each of the four regions. In this case, the platelet cluster is reclassified into four subclusters from the largest particle size: a giant platelet subcluster (PC3), a large platelet subcluster (PC2), a normal platelet subcluster (PC1), and a small platelet subcluster (PC0).

Here, since the horizontal axis of the two-dimensional plot diagram shown in FIG. 8B shows the intensity of the first fluorescence (FL1), which is an indicator of the RNA amount (in other words, immaturity) in each particle, information on the immaturity and information on the size of each particle included in the platelet cluster can be simultaneously obtained based on the two-dimensional plot diagram showing the platelet cluster (PLTn) shown in FIG. 8B. As a result, it opens up the possibility of obtaining clinically useful findings that cannot be grasped from only one of the information (the value of the immature platelet fraction (IPF)).

FIG. 8C is a two-dimensional plot diagram in which, for the nucleated cell cluster (NCn), the intensity of the first fluorescence (FL1) of each particle included in the cluster is the horizontal axis and the size (in this case, the intensity of the forward-scattered light (FS)) of each particle included in the cluster is the vertical axis. Then, in the two-dimensional plot diagram, the vertical axis (intensity of forward-scattered light (FS)) is divided into a plurality of regions (in this case, four regions NC0 to NC3 from the smallest to the largest). Therefore, nucleated cell clusters can be reclassified into a plurality of subclusters based on the number or ratio of particles in each of the four regions. In this case, the nucleated cell cluster is reclassified into four subclusters from the largest particle size: a large nucleated cell subcluster (NC3), a normal nucleated cell subcluster (NC2), a disrupted nucleated cell subcluster (NC1), and a small nucleated cell subcluster (NC0). The information on the immaturity and the information on the size of each particle included in the nucleated cell cluster can be simultaneously obtained based on the two-dimensional plot diagram showing the nucleated cell cluster (NCn) shown in FIG. 8C. As a result, it opens up the possibility of obtaining clinically useful findings that cannot be grasped from only one of the information.

A total number of extracellular vesicles (EV) can be grasped from, for example, a total number of particles in the small erythrocyte subcluster (RC0), the small platelet subcluster (PC0), and the small nucleated cell subcluster (NC0), based on the results shown in the above-mentioned two-dimensional plot diagram. Since information on a distribution of immaturity of EV grasped at that time can be obtained at the same time, clinically useful findings can be provided. The two-dimensional plot diagrams shown in FIG. 8A to FIG. 8C display the events corresponding to the particles included in the erythrocyte cluster, the platelet cluster, and the nucleated cell cluster, respectively. However, as in the Examples described later, the events corresponding to the particles included in each of the plurality of particle clusters may be combined and displayed in one two-dimensional plot diagram.

Another aspect (second aspect) of the present invention also provides a particle analysis method of analyzing particles contained in a blood sample. The particle analysis method according to the second aspect is common with the above-described aspect (first aspect) of the present invention in the following points. That is, the particle analysis method according to the second aspect includes staining the particles with a metachromatic orthochromatic dye, irradiating the stained particles with light, measuring intensity of a first fluorescence derived from a stacking component of the metachromatic orthochromatic dye and intensity of a second fluorescence derived from an intercalation component of the metachromatic orthochromatic dye, the first fluorescence and the second fluorescence being emitted by each particle contained in the blood sample, normalizing the intensity of the first fluorescence and the intensity of the second fluorescence, emitted by each of the particles, by the size of each of the particles to obtain a fluorescence concentration of each of the first fluorescence and the second fluorescence in each of the particles, and clustering each of the particles into a plurality of particle clusters including at least two of an erythrocyte cluster, a platelet cluster and a nucleated cell cluster, in a two-dimensional plot of the fluorescence concentration obtained by the normalization. On the other hand, the particle analysis method according to the second aspect is characterized in that, for at least one particle cluster included in the plurality of particle clusters, a fluorescence intensity ratio that is a ratio of the intensities of the first fluorescence and the second fluorescence of each particle included in the particle cluster is calculated, and a fluorescence intensity ratio histogram in which the fluorescence intensity ratio is a class is created. Hereinafter, a preferred embodiment for carrying out the particle analysis method according to the second aspect will be described with a focus on points different from those of the first aspect.

As described above, also in the particle analysis method according to the second aspect, clustering particles contained in the blood sample into the plurality of particle clusters using an RNP diagram as shown in FIG. 6 is common with the particle analysis method according to the first aspect.

Subsequently, in the particle analysis method according to the second aspect, for at least one particle cluster included in the plurality of particle clusters, the fluorescence intensity ratio that is the ratio of the intensities of the first fluorescence and the second fluorescence of each particle included in the particle cluster is calculated. For example, the erythrocyte cluster (RBCn) is gated from the RNP diagram shown in FIG. 6, and for each particle included in the erythrocyte cluster (RBCn), a ratio of the intensity of the second fluorescence (FL2) to the intensity of the first fluorescence (FL1) (fluorescence intensity ratio (FL2/FL1)) is calculated. Then, a histogram (fluorescence intensity ratio histogram) is created in which each particle included in the erythrocyte cluster (RBCn) is used as an element and the fluorescence intensity ratio (FL2/FL1) is a class.

It is considered that the fluorescence intensity ratio (FL2/FL1) of each particle calculated in this way substantially reflects a ratio of an amount of DNA to an amount of RNA (DNA amount/RNA amount) in each particle. Here, in normal erythrocytes, both the DNA amount and the RNA amount in the cell are not so large. Therefore, for example, in a particle in which the value of the fluorescence intensity ratio (FL2/FL1) calculated as described above is larger than a predetermined threshold value (for example, 2.0), it is conceivable that the DNA amount in the particle is so large with respect to the RNA amount that it deviates from a normal range. Thus, for such particles included in the erythrocyte cluster, the "abnormalities" described in the first aspect, that is, various bodies (such as Howell-Jolly body and Pappenheimer body), malaria parasite, Babesia, Theileria, Trypanosoma, and microphilia of filaria, and the like are suspected to be present. Alternatively, the particles are suspected to be nucleated erythrocytes (NRBC). In this way, the particles included in the blood sample can be analyzed based on the fluorescence intensity ratio histogram created above, and such an analysis also provides clinically useful findings. It goes without saying that the same analysis can be performed even when the fluorescence intensity ratio is a ratio of the intensity of the first fluorescence (FL1) to the intensity of the second fluorescence (FL2) (FL1/FL2). The fluorescence concentrations (CRc and CDc) in each particle calculated in the first aspect are each obtained by dividing the intensity of the first fluorescence (FL1) and the intensity of the second fluorescence (FL2) by the intensity of the forward-scattered light (FS) or the diameter calculated based on the intensity of the forward-scattered light (FS). Therefore, in calculating the fluorescence intensity ratio in the second aspect, even when a ratio of the fluorescence concentration (CRc) of the first fluorescence and the fluorescence concentration (CDc) of the second fluorescence is used instead of the ratio of the intensity of the first fluorescence (FL1) and the intensity of the second fluorescence (FL2), the same value can be obtained.

To summarize the above, a preferred embodiment of the particle analysis method according to the second aspect further includes measuring the number or ratio of particles in which the fluorescence intensity ratio is higher than a predetermined threshold value (for example, 2.0) for the fluorescence intensity ratio when the fluorescence intensity ratio is a value of a ratio of the intensity of the second fluorescence to the intensity of the first fluorescence (FL2/FL1), or measuring the number or ratio of particles in which the fluorescence intensity ratio is smaller than the predetermined threshold value (for example, 0.5) for the fluorescence intensity ratio when the fluorescence intensity ratio is a value of a ratio of the intensity of the first fluorescence to the intensity of the second fluorescence (FL1/FL2), and determining the presence or absence of an abnormality in the particles included in the particle cluster based on a result of the measurement. The specific example of the "abnormality" whose presence is suspected in this case is as described above.

### Examples

Hereinafter, embodiments of the present invention will be specifically described with reference to Examples.

### <<Measurement example to which the first aspect of the present invention is applied>>

### ([Example of measurement of reticulocyte ratio in blood sample without morphological findings]

The reticulocyte fraction was measured for a blood sample collected from an adult male. In a blood morphology test of the blood sample by microscopic observation, no morphological findings regarding erythrocytes and platelets were found. Here, a standard value of the reticulocyte fraction in males is 0.76 to 2.18%.

First, as a control section, a commercially available multi-item automatic blood cell analyzer (XN-series manufactured by Sysmex Corporation) was used, and the reticulocyte fraction was measured according to the attached manual. As a result, the result was obtained that the reticulocyte fraction in the blood sample was 2.7%.

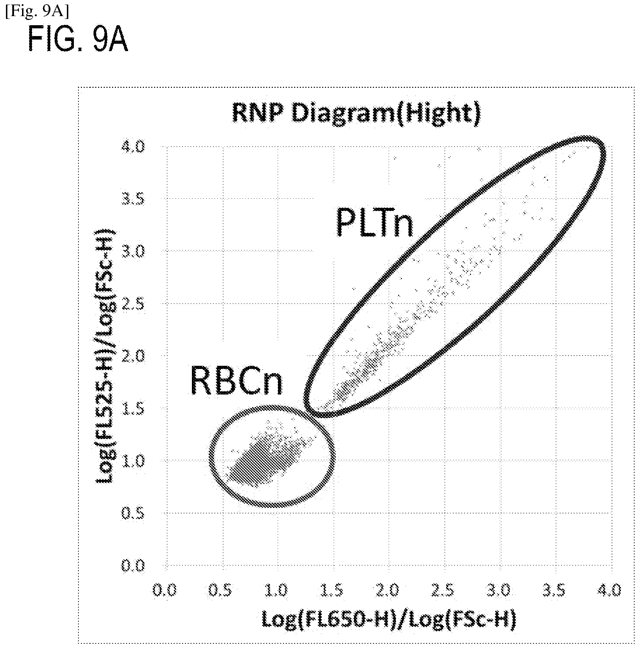

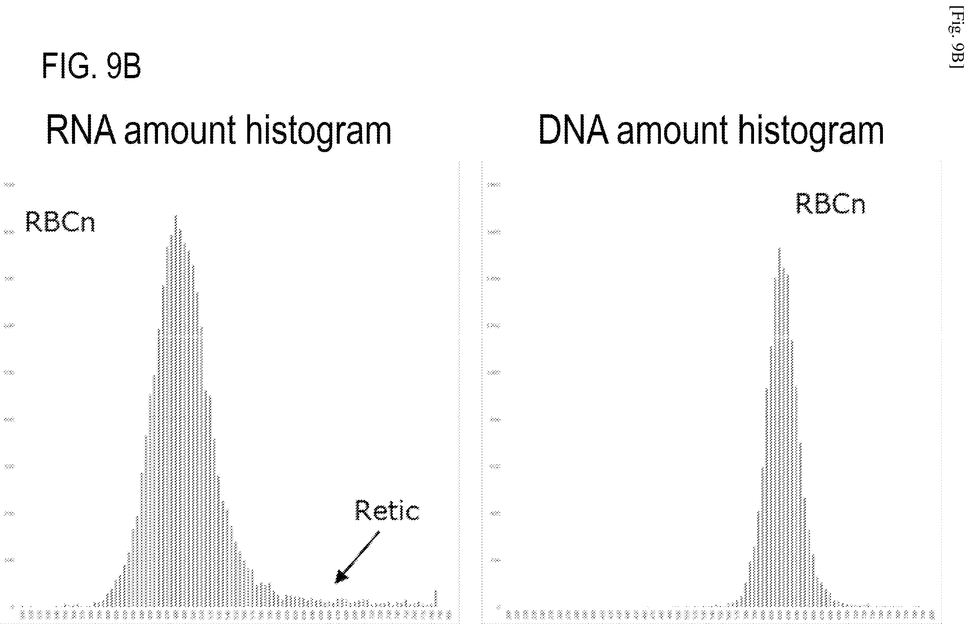

On the other hand, as a measurement example to which the first aspect of the present invention is applied, first, the same blood sample as above was provided, and 5 µL of the blood sample was added to 2 mL of a 0.006 g/L solution of acridine orange (AO), which is a metachromatic orthochromatic dye, and mixed to prepare a measurement sample. Then, measurement was performed using a fully automatic blood cell counter (manufactured by Nihon Kohden Corporation, MEK-9000 series, Celltac G + prototype). Next, using data of obtained FS, SS, FL1 (fluorescence wavelength of 525 nm) and FL2 (fluorescence wavelength of 650 nm), an RNP diagram as shown in FIG. 6 was created. Here, the RNP diagram actually created using the above blood sample is shown in FIG. 9A. Then, the data was separated from the RNP diagram by setting (gating) a gate to the erythrocyte cluster, and an RNA amount histogram and a DNA amount histogram as shown in FIG. 7A were created for the particles included in the separated erythrocyte cluster. Here, FIG. 9B shows the RNA amount histogram (left in FIG. 9B) and the DNA amount histogram (right in FIG. 9B) actually created using the blood sample. Then, a threshold value was set for the horizontal axis (intensity of the first fluorescence (FL1) reflecting the RNA amount) based on the peak of the RNA amount histogram, and particles in which a value on the horizontal axis was equal to or more than the threshold value were determined as reticulocytes (Retic). Then, when the reticulocyte fraction was calculated as a ratio of reticulocytes occupying the particles included in the erythrocyte cluster, the reticulocyte fraction in the blood sample was 2.97%. As described above, in the blood sample in which no morphological findings were found for erythrocytes and platelets, when the first aspect of the present invention was applied, a value of reticulocyte fraction almost equal to that in the control section was obtained.

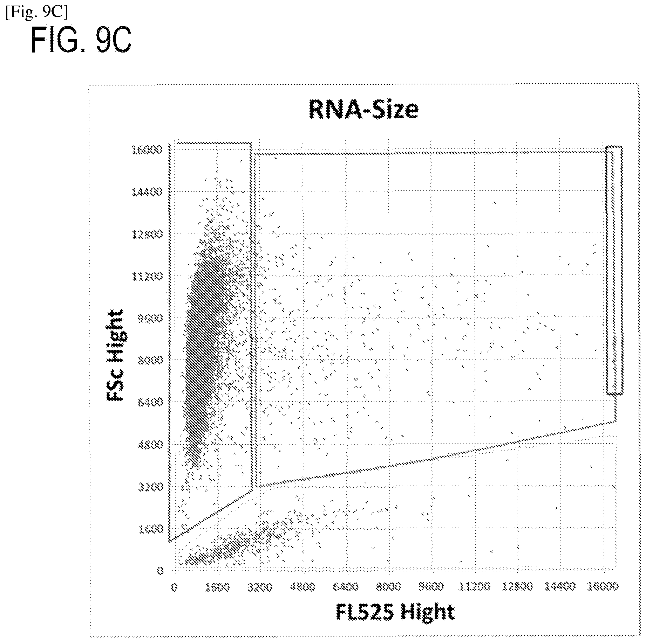

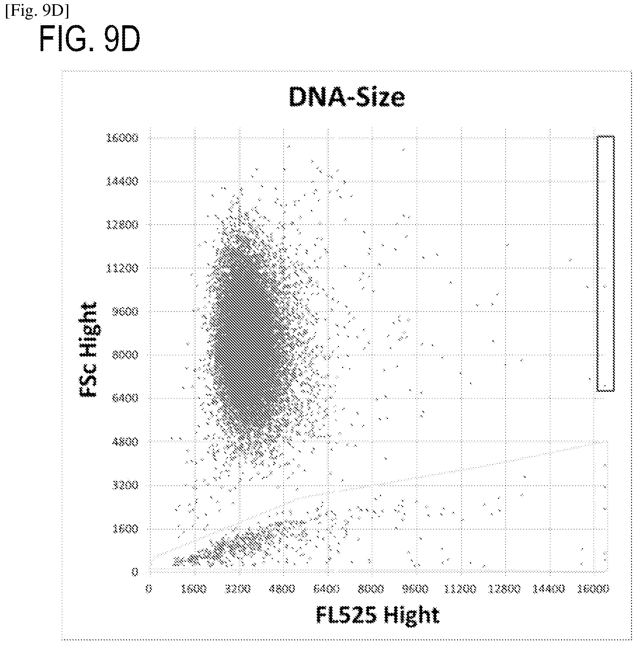

Based on the RNP diagram (FIG. 9A), for the erythrocyte cluster in which the RNA amount histogram (left in FIG. 9B) and the DNA amount histogram (right in FIG. 9B) were created above, a two-dimensional plot diagram was created in which the intensity of the first fluorescence (FL1) of each particle included in the erythrocyte cluster was the horizontal axis and the size of each particle included in the erythrocyte cluster was the vertical axis (FIG. 9C). Similarly, a two-dimensional plot diagram was created in which the intensity of the second fluorescence (FL2) of each particle included in the erythrocyte cluster was the horizontal axis and the size of each particle included in the erythrocyte cluster was the vertical axis (FIG. 9D). In the two-dimensional plot diagrams shown in FIG. 9C and FIG. 9D, the intensity of the forward-scattered light (FS) was adopted for the vertical axis (size of each particle). In these two-dimensional plot diagrams, in addition to the event of the particles included in the erythrocyte cluster, the event of the particles gated from the data of the RNP diagram in the same manner as above for the particles included in the platelet cluster is also displayed together in different colors.

### (Example of measurement of reticulocyte fraction in blood sample with morphological findings of erythrocyte)

The reticulocyte fraction was measured for a blood sample collected from an adult male. In the blood morphology test of the blood sample by microscopic observation, morphological findings regarding erythrocytes were found. Specifically, 17 nucleated erythrocytes (NRBC) were counted in 100 white blood cell counts. The presence of Howell-Jolly body (HJ body) was confirmed in more than half of erythrocyte cells, and the presence of Pappenheimer body (PH body) was also confirmed in some erythrocyte cells.

First, as a control section, a commercially available multi-item automatic blood cell analyzer (XN-series manufactured by Sysmex Corporation) was used, and the reticulocyte fraction was measured according to the attached manual. As a result, the result was obtained that the reticulocyte fraction in the blood sample was 7.97%.

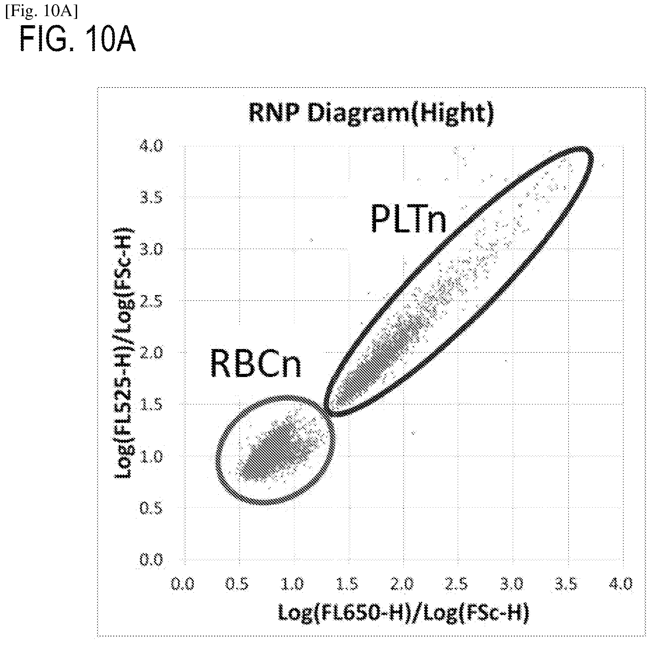

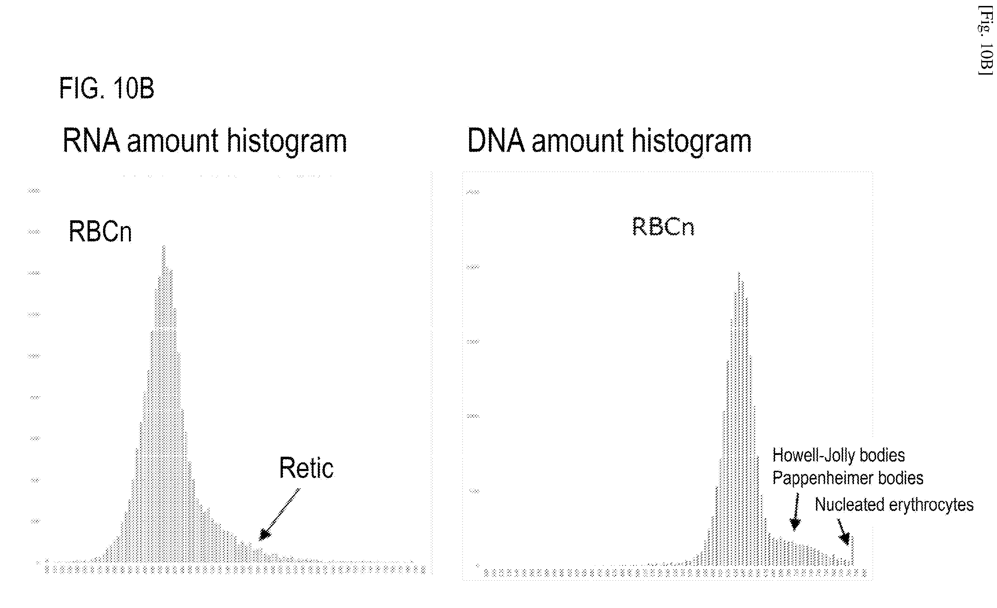

On the other hand, as a measurement example to which the first aspect of the present invention is applied, first, the same blood sample as above was provided, and 5 µL of the blood sample was added to 2 mL of a 0.006 g/L solution of acridine orange (AO), which is a metachromatic orthochromatic dye, and mixed to prepare a measurement sample. Then, measurement was performed using a fully automatic blood cell counter (manufactured by Nihon Kohden Corporation, MEK-9000 series, Celltac G + prototype). Next, using data of obtained FS, SS, FL1 (fluorescence wavelength of 525 nm) and FL2 (fluorescence wavelength of 650 nm), an RNP diagram as shown in FIG. 6 was created in the same manner as above. Here, the RNP diagram actually created using the above blood sample is shown in FIG. 10A. Then, the data was separated from the RNP diagram by setting (gating) a gate to the erythrocyte cluster, and an RNA amount histogram and a DNA amount histogram as shown in FIG. 7A were created for the particles included in the separated erythrocyte cluster. Here, FIG. 10B shows the RNA amount histogram (left in FIG. 10B) and the DNA amount histogram (right in FIG. 10B) actually created using the blood sample. Then, a threshold value was set for the horizontal axis (intensity of the first fluorescence (FL1) reflecting the RNA amount) based on the peak of the RNA amount histogram, and particles in which a value on the horizontal axis was equal to or more than the threshold value were determined as reticulocytes (Retic). Then, when the reticulocyte fraction was calculated as a ratio of reticulocytes occupying the particles included in the erythrocyte cluster, the reticulocyte fraction in the blood sample was 2.97%. As described above, as a result of the measurement of the reticulocyte fraction for the blood sample in which morphological findings regarding erythrocytes were found, when the first aspect of the present invention was applied, a value close to the standard value was obtained, and, on the other hand, in the control section, a value with a large deviation was obtained. Here, in the measurement method of the control section, the fluorescence emitted by the particles is a single color without using the metachromatic orthochromatic dye. Thus, it is not possible to distinguish and detect between the RNA-derived stacking component and the DNA-derived intercalation component. As a result, in the application example of the present invention, the presence of various bodies and nucleated erythrocytes shown on the right in FIG. 10B, which can be detected to be distinguished from reticulocytes, is erroneously included in reticulocytes in the control section and counted, so that it is considered that the measured value of the reticulocyte fraction deviates greatly from the actual value. When the measured value of the reticulocyte fraction deviates greatly from the actual value, there is a problem that the presence of diseases (for example, hemolytic anemia, iron deficiency anemia, pernicious anemia) in which the reticulocyte fraction is high is suspected.

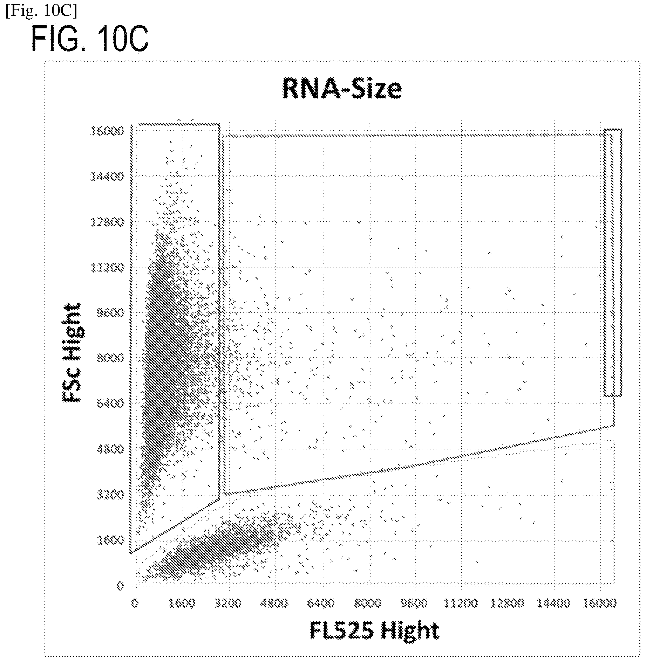

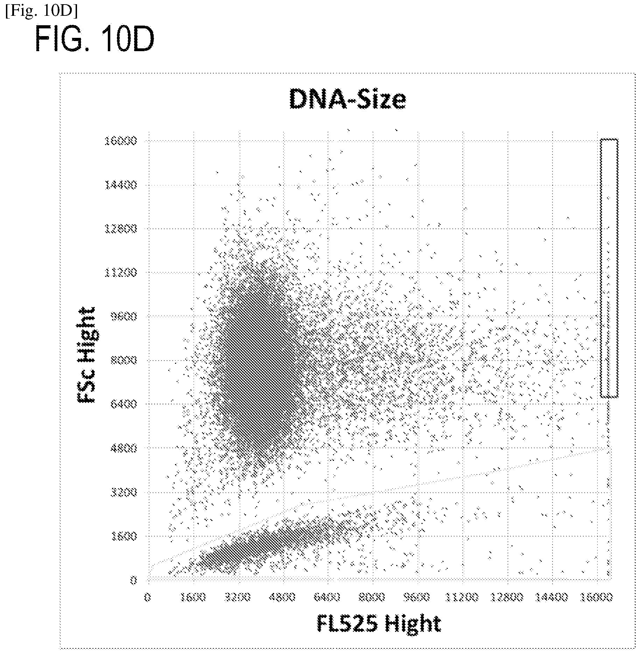

Based on the RNP diagram (FIG. 10A), for the erythrocyte cluster in which the RNA amount histogram (left in FIG. 10B) and the DNA amount histogram (right in FIG. 10B) were created above, a two-dimensional plot diagram was created in which the intensity of the first fluorescence (FL1) of each particle included in the erythrocyte cluster was the horizontal axis and the size of each particle included in the erythrocyte cluster was the vertical axis (FIG. 10C). Similarly, a two-dimensional plot diagram was created in which the intensity of the second fluorescence (FL2) of each particle included in the erythrocyte cluster was the horizontal axis and the size of each particle included in the erythrocyte cluster was the vertical axis (FIG. 10D). In the two-dimensional plot diagrams shown in FIG. 10C and FIG. 10D, the intensity of the forward-scattered light (FS) was adopted for the vertical axis (size of each particle). In these two-dimensional plot diagrams, in addition to the event of the particles included in the erythrocyte cluster, the event of the particles gated from the data of the RNP diagram in the same manner as above for the particles included in the platelet cluster is also displayed together in different colors. Here, for example, in the two-dimensional plot diagram of FS × FL2 shown in FIG. 10D, the event of the erythrocyte cluster is distributed so as to spread in a horizontal axis direction as compared with the two-dimensional plot diagram of FS × FL2 shown in FIG. 9D. This corresponds to various bodies and nucleated erythrocytes whose presence has been confirmed in the DNA amount histogram shown on the right in FIG. 10B. Comparing the two-dimensional plot diagrams shown in FIG. 9D and FIG. 10D, it can be seen that the size of the particles included in the erythrocyte cluster is relatively small in the blood sample with morphological findings of erythrocytes, and it can also be seen that the number of particles included in the platelet cluster is relatively large, for example.

### <<Measurement example to which the second aspect of the present invention is applied>>

### (Example of measurement of blood sample without morphological findings)

A blood sample collected from an adult male was measured by applying the second aspect of the present invention. In a blood morphology test of the blood sample by microscopic observation, no morphological findings regarding erythrocytes and platelets were found.

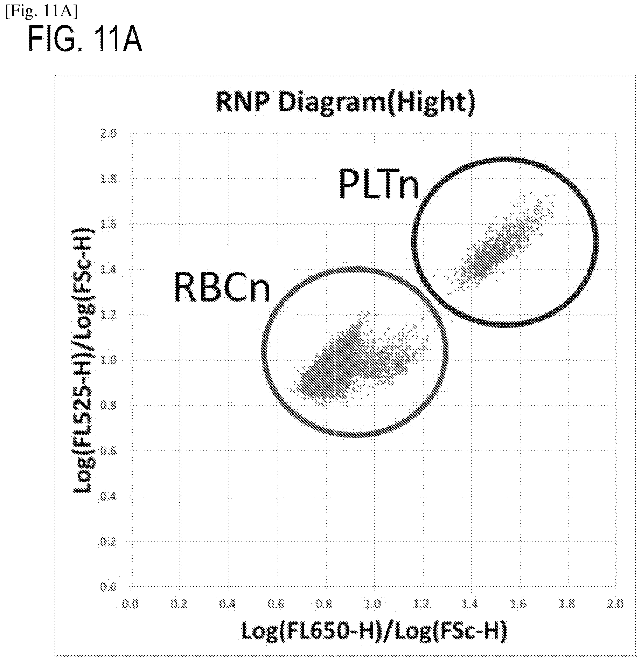

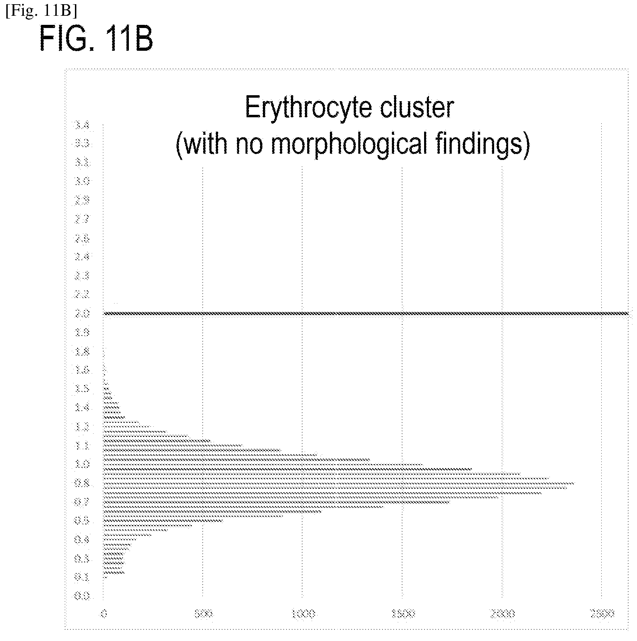

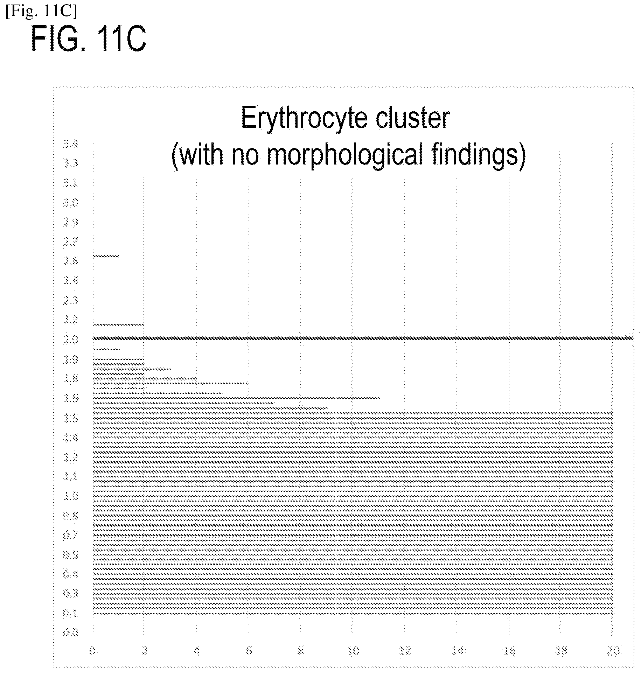

First, 5 µL of the blood sample provided above was added to 2 mL of a 0.006 g/L solution of acridine orange (AO), which was a metachromatic orthochromatic dye, and mixed to prepare a measurement sample. Then, measurement was performed using a fully automatic blood cell counter (manufactured by Nihon Kohden Corporation, MEK-9000 series, Celltac G + prototype). Next, using data of obtained FS, SS, FL1 (fluorescence wavelength of 525 nm) and FL2 (fluorescence wavelength of 650 nm), an RNP diagram as shown in FIG. 6 was created. Here, the RNP diagram actually created using the above blood sample is shown in FIG. 11A. Then, data was separated from the RNP diagram by setting (gating) a gate to the erythrocyte cluster. For the particles included in the separated erythrocyte cluster, the fluorescence intensity ratio, which was the value of the ratio of the intensity of the first fluorescence (FL1) to the intensity of the second fluorescence (FL2) (FL1/FL2) of each particle, was calculated, and the fluorescence intensity ratio histogram in which the fluorescence intensity ratio was a class was created. Here, the fluorescence intensity ratio histogram actually created using the above blood sample is shown in FIG. 11B. In the fluorescence intensity ratio histogram shown in FIG. 11B, a threshold value that is an indicator of an abnormal value of the fluorescence intensity ratio is set to FL1/FL2 = 2.0. FIG. 11C is an enlarged view of a region near the threshold value (FL1/FL2 = 2.0) in FIG. 11B. According to the fluorescence intensity ratio histograms shown in FIG. 11B and FIG. 11C, the number of particles in which the fluorescence intensity ratio was higher than 2.0 that was the threshold value was three. This result is consistent with the absence of morphological findings regarding erythrocytes in the blood morphology test.

### (Example of measurement of blood sample with morphological findings of erythrocytes (1))

A blood sample collected from an adult male was measured by applying the second aspect of the present invention. In the blood morphology test of the blood sample by microscopic observation, morphological findings regarding erythrocytes were found. Specifically, the presence of Howell-Jolly body (HJ body) was confirmed in erythrocyte cells.

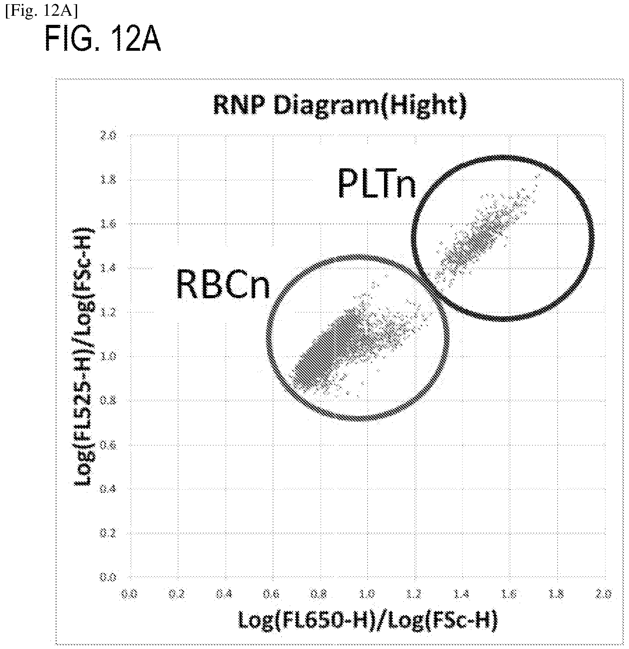

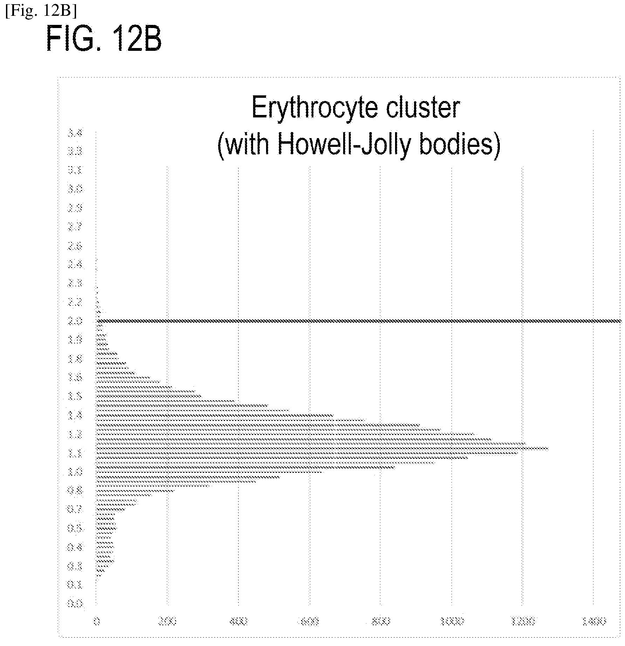

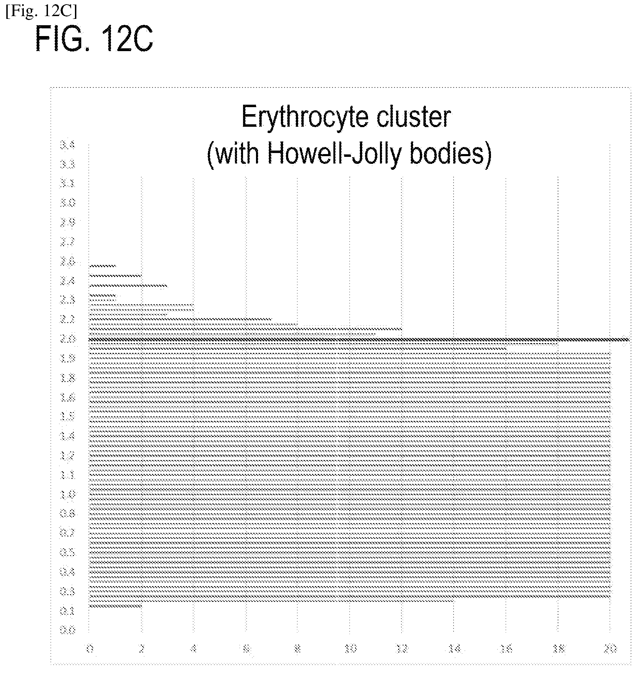

Using this blood sample, a measurement sample was prepared by the same method as above, and measurement was performed using a fully automatic blood cell counter (manufactured by Nihon Kohden Corporation, MEK-9000 series, Celltac G + prototype). Next, using data of obtained FS, SS, FL1 (fluorescence wavelength of 525 nm) and FL2 (fluorescence wavelength of 650 nm), an RNP diagram as shown in FIG. 6 was created. Here, the RNP diagram actually created using the above blood sample is shown in FIG. 12A. Then, data was separated from the RNP diagram by setting (gating) a gate to the erythrocyte cluster. For the particles included in the separated erythrocyte cluster, the fluorescence intensity ratio, which was the value of the ratio of the intensity of the first fluorescence (FL1) to the intensity of the second fluorescence (FL2) (FL1/FL2) of each particle, was calculated, and the fluorescence intensity ratio histogram in which the fluorescence intensity ratio was a class was created. Here, the fluorescence intensity ratio histogram actually created using the above blood sample is shown in FIG. 12B. In the fluorescence intensity ratio histogram shown in FIG. 12B, a threshold value that is an indicator of the abnormal value of the fluorescence intensity ratio is set to FL1/FL2 = 2.0. FIG. 12C is an enlarged view of the region near the threshold value (FL1/FL2 = 2.0) in FIG. 12B. According to the fluorescence intensity ratio histograms shown in FIG. 12B and FIG. 12C, 57 particles in which the fluorescence intensity ratio was higher than 2.0 that was the threshold value were counted, and their fluorescence intensity ratios were concentrated in a region slightly higher than 2.0. This result is consistent with the observation of the presence of the HJ body in the blood morphology test.

### (Example of measurement of blood sample with morphological findings of erythrocytes (2))

A blood sample collected from an adult male was measured by applying the second aspect of the present invention. In the blood morphology test of the blood sample by microscopic observation, morphological findings regarding erythrocytes were found. Specifically, the appearance of the nucleated erythrocyte (NRBC) was confirmed in 100 white blood cell counts.

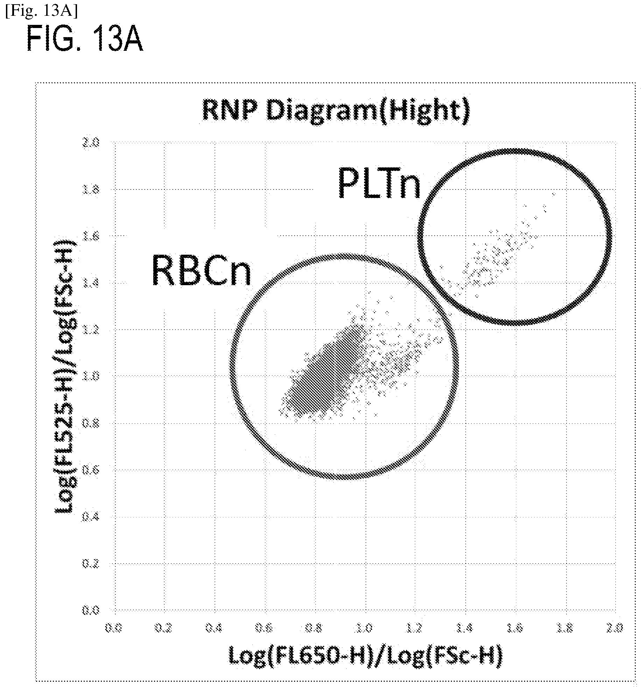

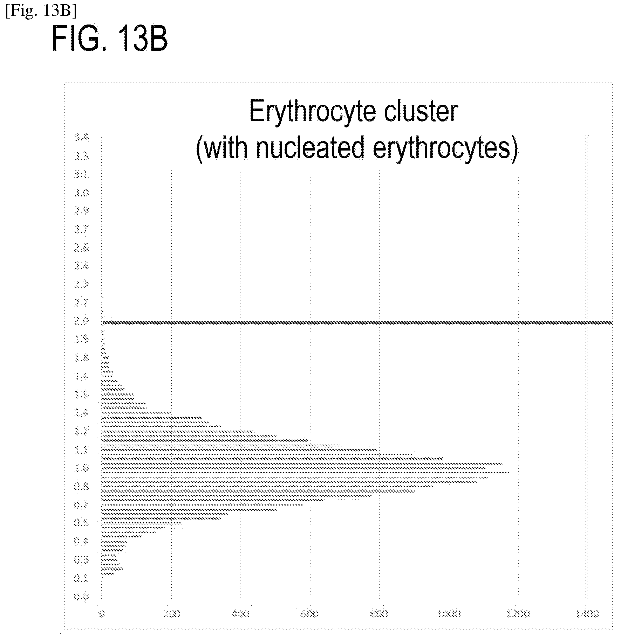

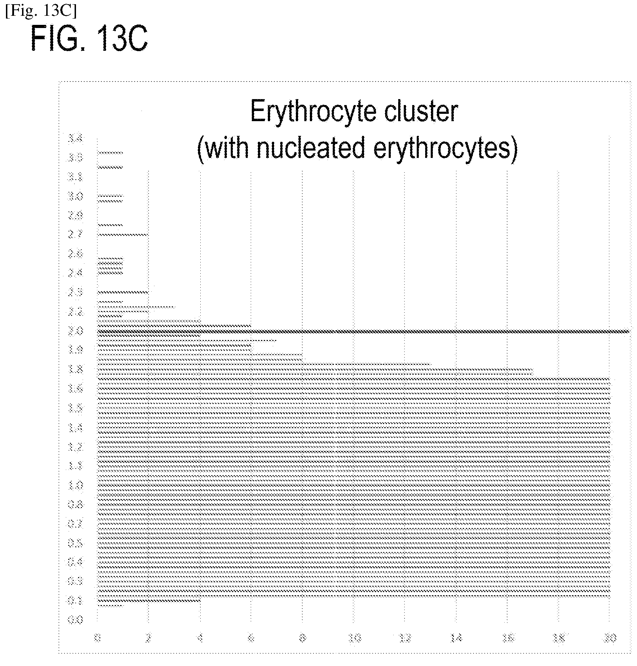

Using this blood sample, a measurement sample was prepared by the same method as above, and measurement was performed using a fully automatic blood cell counter (manufactured by Nihon Kohden Corporation, MEK-9000 series, Celltac G + prototype). Next, using data of obtained FS, SS, FL1 (fluorescence wavelength of 525 nm) and FL2 (fluorescence wavelength of 650 nm), an RNP diagram as shown in FIG. 6 was created. Here, the RNP diagram actually created using the above blood sample is shown in FIG. 13A. Then, data was separated from the RNP diagram by setting (gating) a gate to the erythrocyte cluster. For the particles included in the separated erythrocyte cluster, the fluorescence intensity ratio, which was the value of the ratio of the intensity of the first fluorescence (FL1) to the intensity of the second fluorescence (FL2) (FL1/FL2) of each particle, was calculated, and the fluorescence intensity ratio histogram in which the fluorescence intensity ratio was a class was created. Here, the fluorescence intensity ratio histogram actually created using the above blood sample is shown in FIG. 13B. In the fluorescence intensity ratio histogram shown in FIG. 13B, a threshold value that is an indicator of the abnormal value of the fluorescence intensity ratio is set to FL1/FL2 = 2.0. FIG. 13C is an enlarged view of the region near the threshold value (FL1/FL2 = 2.0) in FIG. 13B. According to the fluorescence intensity ratio histograms shown in FIG. 13B and FIG. 13C, 25 particles in which the fluorescence intensity ratio was higher than 2.0 that was the threshold value were counted, and their fluorescence intensity ratios were widely distributed in a region of 2.0 to 3.3. This result is consistent with the observation of the presence of the nucleated erythrocyte (NRBC) in the blood morphology test.

As described above, the particles in the blood sample are analyzed by applying the present invention using the metachromatic orthochromatic dye, so that more clinically useful information can be obtained.

### Reference Signs List

10 Metachromatic orthochromatic dye
10A Dispensed metachromatic orthochromatic dye
20 Blood sample
30 Measurement sample
40 Sample preparation unit
50 Flow cytometer
51 Flow cell
52 Laser light source
53 Irradiation-light condensing lens
54 Scattered-light condensing lens
55, 56, 57 Beam splitter
58, 59 Wavelength selection filter
61 Detector for small-angled forward-scattered light (FSs)
62 Detector for large-angled forward-scattered light (FLs)
63 Lateral-scattered light detector (SS)
64 First fluorescence detector (FL1)
65 Second fluorescence detector (FL2)
70 Processor (CPU)

## Claims

1. A particle analysis method of analyzing particles contained in a blood sample (20), comprising:
staining the particles with a metachromatic orthochromatic dye (10, 10A) ;
irradiating the stained particles with light;
measuring intensity of a first fluorescence derived from a stacking component of the metachromatic orthochromatic dye (10, 10A) and intensity of a second fluorescence derived from an intercalation component of the metachromatic orthochromatic dye (10, 10A), the first fluorescence and the second fluorescence being emitted by each particle contained in the blood sample (20);
normalizing the intensity of the first fluorescence and the intensity of the second fluorescence, emitted by each of the particles, by the size of each of the particles to obtain a fluorescence concentration of each of the first fluorescence and the second fluorescence in each of the particles;
clustering each of the particles into a plurality of particle clusters including at least two of an erythrocyte cluster, a platelet cluster and a nucleated cell cluster, in a two-dimensional plot of the fluorescence concentration obtained by the normalization;
creating an RNA amount histogram in which the intensity of the first fluorescence is a class and a DNA amount histogram in which the intensity of the second fluorescence is a class for at least one particle cluster included in the plurality of particle clusters; and
for the at least one particle cluster in which the RNA amount histogram and the DNA amount histogram are created, creating a two-dimensional plot diagram in which the intensity of the first fluorescence or the intensity of the second fluorescence of each particle included in the particle cluster is one axis and the size of each particle included in the particle cluster is the other axis.

2. The particle analysis method according to claim 1, further comprising analyzing the particles contained in the blood sample (20) based on the RNA amount histogram and the DNA amount histogram.

3. The particle analysis method according to claim 2, comprising creating the RNA amount histogram and the DNA amount histogram for the erythrocyte cluster,
wherein the analysis includes one or more of:
(a) measuring the number or ratio of reticulocytes contained in the blood sample (20) and/or an immature reticulocyte fraction (IRF) of the blood sample (20) based on the RNA amount histogram of the erythrocyte cluster;
(b) determining a presence or absence of an abnormality in the erythrocyte cluster based on the DNA amount histogram of the erythrocyte cluster;
(c) measuring an immature platelet fraction (IPF) in the blood sample (20) based on the RNA amount histogram of the platelet cluster; and
(d) determining a presence or absence of an abnormality in the platelet cluster based on the DNA amount histogram of the platelet cluster.

4. The particle analysis method according to claim 3, wherein the abnormality is a presence of a body, malaria parasite, Babesia, Theileria, Trypanosoma, or microphilia of filaria in the particles included in the particle cluster.

5. The particle analysis method according to claim 1, further comprising dividing an axis indicating the size of each of the particles in the two-dimensional plot diagram into a plurality of regions and reclassifying the at least one particle cluster into a plurality of subclusters based on the number or ratio of the particles in each of the plurality of regions formed by the division;
optionally wherein the two-dimensional plot diagram includes a two-dimensional plot diagram for an erythrocyte cluster, the particle analysis method further comprising reclassifying the erythrocyte cluster into a plurality of subclusters including a subcluster of normal erythrocytes, a large erythrocyte subcluster, a disrupted erythrocyte subcluster, and/or a small erythrocyte subcluster.

6. The particle analysis method according to claim 5, wherein the two-dimensional plot diagram includes a two-dimensional plot diagram for a platelet cluster, the particle analysis method further comprising reclassifying the platelet cluster into a plurality of subclusters including a subcluster of normal platelets, a giant platelet subcluster, a large platelet subcluster, and/or a small platelet subcluster.

7. The particle analysis method according to any one of claims 5 or 6, wherein the two-dimensional plot diagram includes a two-dimensional plot diagram for a nucleated cell cluster, the particle analysis method further comprising reclassifying the nucleated cell cluster into a plurality of subclusters including a subcluster of normal nucleated cells, a large nucleated cell subcluster, a disrupted nucleated cell subcluster, and/or a small nucleated cell subcluster.

8. The particle analysis method according to any one of claims 1 to 7, wherein the first fluorescence is an orange fluorescence, and the second fluorescence is a green fluorescence, optionally wherein the metachromatic orthochromatic dye (10, 10A) is acridine orange (A0).

9. The particle analysis method according to any one of claims 1 to 7, wherein a central wavelength of the light applied to the particles contained in the blood sample (20) is 408 nm, 445 nm, 473 nm or 488 nm.

10. A particle analyzer comprising:
a light source (52) that applies light to particles contained in a blood sample (20);
a flow cell (51) through which the blood sample (20) flows;
a light detector including a plurality of fluorescence detectors (61, 62, 63, 64, 65) that detect each of intensity of a first fluorescence and intensity of a second fluorescence having different wavelengths; and
a data processing part (70) that:
normalizes the intensities of the first fluorescence and the second fluorescence emitted by each of the particles contained in the blood sample (20) by the size of each of the particles to determine each fluorescence concentration of the first fluorescence and the second fluorescence in each of the particles;
clusters each of the particles into a plurality of particle clusters including at least two of an erythrocyte cluster, a platelet cluster and a nucleated cell cluster, in a two-dimensional plot of the fluorescence concentration obtained by the normalization;
creates an RNA amount histogram in which the intensity of the first fluorescence is a class and a DNA amount histogram in which the intensity of the second fluorescence is a class for at least one particle cluster included in the plurality of particle clusters; and
for the at least one particle cluster in which the RNA amount histogram and the DNA amount histogram are created, creates a two-dimensional plot diagram in which the intensity of the first fluorescence or the intensity of the second fluorescence of each particle included in the particle cluster is one axis and the size of each particle included in the particle cluster is the other axis.

11. The particle analyzer according to claim 10, wherein the data processing part (70) analyzes the particles contained in the blood sample (20) based on the RNA amount histogram and the DNA amount histogram.

## Patentansprüche

1. Teilchenanalyseverfahren zum Analysieren von Teilchen, die in einer Blutprobe (20) enthalten sind, umfassend:
Anfärben der Teilchen mit einem metachromatischen orthochromatischen Farbstoff (10, 10A);
Bestrahlen der angefärbten Teilchen mit Licht;
Messen einer Intensität einer ersten Fluoreszenz, die von einer Stapelkomponente des metachromatischen orthochromatischen Farbstoffs (10, 10A) stammt, und einer Intensität einer zweiten Fluoreszenz, die von einer Interkalationskomponente des metachromatischen orthochromatischen Farbstoffs (10, 10A) stammt, wobei die erste Fluoreszenz und die zweite Fluoreszenz von jedem in der Blutprobe (20) enthaltenen Teilchen emittiert werden;
Normieren der Intensität der ersten Fluoreszenz und der Intensität der zweiten Fluoreszenz, die von jedem der Teilchen emittiert werden, durch die Größe jedes der Teilchen, um eine Fluoreszenzkonzentration jeder der ersten Fluoreszenz und der zweiten Fluoreszenz in jedem der Teilchen zu erhalten;
Clustern jedes der Teilchen in eine Vielzahl von Teilchenclustern, die mindestens zwei von einem Erythrozytencluster, einem Thrombozytencluster und einem kernhaltigen Zellcluster einschließen, in einem zweidimensionalen Verlauf der durch die Normierung erhaltenen Fluoreszenzkonzentration;
Erstellen eines RNA-Mengenhistogramms, in dem die Intensität der ersten Fluoreszenz eine Klasse ist, und eines DNA-Mengenhistogramms, in dem die Intensität der zweiten Fluoreszenz eine Klasse ist, für mindestens einen Teilchencluster, der in der Vielzahl von Teilchenclustern eingeschlossen ist; und
Erstellen eines zweidimensionalen Verlaufsdiagramms, in dem die Intensität der ersten Fluoreszenz oder die Intensität der zweiten Fluoreszenz jedes in dem Teilchencluster eingeschlossenen Teilchens eine Achse ist und die Größe jedes in dem Teilchencluster eingeschlossenen Teilchens die andere Achse ist, für den mindestens einen Teilchencluster, in dem das RNA-Mengenhistogramm und das DNA-Mengenhistogramm erstellt werden.

2. Teilchenanalyseverfahren nach Anspruch 1, ferner umfassend ein Analysieren der in der Blutprobe (20) enthaltenen Teilchen basierend auf dem RNA-Mengenhistogramm und dem DNA-Mengenhistogramm.

3. Teilchenanalyseverfahren nach Anspruch 2, umfassend ein Erstellen des RNA-Mengenhistogramms und des DNA-Mengenhistogramms für den Erythrozytencluster,
wobei die Analyse eines oder mehrere von Folgendem einschließt:
(a) Messen der Anzahl oder des Anteils von Retikulozyten, die in der Blutprobe (20) enthalten sind, und/oder einer Fraktion unreifer Retikulozyten (IRF) der Blutprobe (20) basierend auf dem RNA-Mengenhistogramm des Erythrozytenclusters;
(b) Bestimmen eines Vorhandenseins oder Nichtvorhandenseins einer Abnormalität in dem Erythrozytencluster basierend auf dem DNA-Mengenhistogramm des Erythrozytenclusters;
(c) Messen einer Fraktion unreifer Thrombozyten (IPF) in der Blutprobe (20) basierend auf dem RNA-Mengenhistogramm des Thrombozytenclusters; und
(d) Bestimmen eines Vorhandenseins oder Nichtvorhandenseins einer Abnormalität in dem Thrombozytencluster basierend auf dem DNA-Mengenhistogramm des Thrombozytenclusters.

4. Teilchenanalyseverfahren nach Anspruch 3, wobei die Abnormalität ein Vorhandensein eines Körpers, eines Malariaparasiten, von Babesien, Theilerien, Trypanosomen oder Mikrofilarien in den in dem Teilchencluster eingeschlossenen Teilchen ist.

5. Teilchenanalyseverfahren nach Anspruch 1, ferner umfassend ein Unterteilen einer Achse, die die Größe jedes der Teilchen in dem zweidimensionalen Verlaufsdiagramm angibt, in eine Vielzahl von Regionen und ein Neuklassifizieren des mindestens einen Teilchenclusters in eine Vielzahl von Subclustern basierend auf der Anzahl oder dem Anteil der Teilchen in jeder der Vielzahl von durch die Unterteilung ausgebildeten Regionen; wobei optional das zweidimensionale Verlaufsdiagramm ein zweidimensionales Verlaufsdiagramm für einen Erythrozytencluster einschließt, wobei das Teilchenanalyseverfahren ferner ein Neuklassifizieren des Erythrozytenclusters in eine Vielzahl von Subclustern umfasst, die einen Subcluster normaler Erythrozyten, einen Subcluster großer Erythrozyten, einen Subcluster unterbrochener Erythrozyten und/oder einen Subcluster kleiner Erythrozyten einschließen.

6. Teilchenanalyseverfahren nach Anspruch 5, wobei das zweidimensionale Verlaufsdiagramm ein zweidimensionales Verlaufsdiagramm für einen Thrombozytencluster einschließt, wobei das Teilchenanalyseverfahren ferner ein Neuklassifizieren des Thrombozytenclusters in eine Vielzahl von Subclustern umfasst, die einen Subcluster normaler Thrombozyten, einen Subcluster riesiger Thrombozyten, einen Subcluster großer Thrombozyten und/oder einen Subcluster kleiner Thrombozyten einschließen.

7. Teilchenanalyseverfahren nach einem der Ansprüche 5 oder 6, wobei das zweidimensionale Verlaufsdiagramm ein zweidimensionales Verlaufsdiagramm für einen kernhaltigen Zellcluster einschließt, wobei das Teilchenanalyseverfahren ferner ein Neuklassifizieren des kernhaltigen Zellclusters in eine Vielzahl von Subclustern umfasst, die einen Subcluster normaler kernhaltiger Zellen, einen Subcluster großer kernhaltiger Zellen, einen Subcluster unterbrochener kernhaltiger Zellen und/oder einen Subcluster kleiner kernhaltiger Zellen einschließen.

8. Teilchenanalyseverfahren nach einem der Ansprüche 1 bis 7, wobei die erste Fluoreszenz eine orange Fluoreszenz ist und die zweite Fluoreszenz eine grüne Fluoreszenz ist, wobei optional der metachromatische orthochromatische Farbstoff (10, 10A) Acridinorange (A0) ist.

9. Teilchenanalyseverfahren nach einem der Ansprüche 1 bis 7, wobei eine zentrale Wellenlänge des auf die in der Blutprobe (20) enthaltenen Teilchen angewendeten Lichts 408 nm, 445 nm, 473 nm oder 488 nm ist.

10. Teilchenanalysator, umfassend:
eine Lichtquelle (52), die Licht auf die in einer Blutprobe (20) enthaltenen Teilchen anwendet;
eine Durchflusszelle (51), durch die die Blutprobe (20) fließt;
einen Lichtdetektor, der eine Vielzahl von Fluoreszenzdetektoren (61, 62, 63, 64, 65) einschließt, die jede einer Intensität einer ersten Fluoreszenz und einer Intensität einer zweiten Fluoreszenz detektieren, die unterschiedliche Wellenlängen aufweisen; und
einen Datenverarbeitungsteil (70), der:
die Intensitäten der ersten Fluoreszenz und der zweiten Fluoreszenz, die durch jedes der in der Blutprobe (20) enthaltenen Teilchen emittiert werden, durch die Größe jedes der Teilchen normiert, um jede Fluoreszenzkonzentration der ersten Fluoreszenz und der zweiten Fluoreszenz in jedem der Teilchen zu bestimmen;
jedes der Teilchen in eine Vielzahl von Teilchenclustern, die mindestens zwei von einem Erythrozytencluster, einem Thrombozytencluster und einem kernhaltigen Zellcluster einschließen, in einem zweidimensionalen Verlauf der durch die Normierung erhaltenen Fluoreszenzkonzentration clustert;
ein RNA-Mengenhistogramm, in dem die Intensität der ersten Fluoreszenz eine Klasse ist, und ein DNA-Mengenhistogramm, in dem die Intensität der zweiten Fluoreszenz eine Klasse ist, für mindestens einen Teilchencluster, der in der Vielzahl von Teilchenclustern eingeschlossen ist, erstellt; und
für den mindestens einen Teilchencluster, in dem das RNA-Mengenhistogramm und das DNA-Mengenhistogramm erstellt werden, ein zweidimensionales Verlaufsdiagramm erstellt, in dem die Intensität der ersten Fluoreszenz oder die Intensität der zweiten Fluoreszenz jedes in dem Teilchencluster eingeschlossenen Teilchens eine Achse ist und die Größe jedes in dem Teilchencluster eingeschlossenen Teilchens die andere Achse ist.

11. Teilchenanalysator nach Anspruch 10, wobei der Datenverarbeitungsteil (70) die in der Blutprobe (20) enthaltenen Teilchen basierend auf dem RNA-Mengenhistogramm und dem DNA-Mengenhistogramm analysiert.

## Revendications

1. Procédé d'analyse de particules pour analyser des particules contenues dans un échantillon de sang (20), comprenant :
la coloration des particules avec un colorant métachromatique orthochromatique (10, 10A) ;
l'irradiation des particules colorées avec de la lumière ;
la mesure d'une intensité d'une première fluorescence dérivée d'un composant d'empilement du colorant métachromatique orthochromatique (10, 10A) et d'une intensité d'une seconde fluorescence dérivée d'un composant d'intercalation du colorant métachromatique orthochromatique (10, 10A), la première fluorescence et la seconde fluorescence étant émises par chaque particule contenue dans l'échantillon de sang (20) ;
la normalisation de l'intensité de la première fluorescence et l'intensité de la seconde fluorescence, émises par chacune des particules, par la taille de chacune des particules pour obtenir une concentration de fluorescence de chacune de la première fluorescence et de la seconde fluorescence dans chacune des particules ;
le groupement de chacune des particules en une pluralité de groupes de particules comportant au moins deux d'un groupe d'érythrocytes, d'un groupe de plaquettes et d'un groupe de cellules nucléées, dans un graphique bidimensionnel de la concentration de fluorescence obtenue par la normalisation ;
la création d'un histogramme de quantité d'ARN dans lequel l'intensité de la première fluorescence est une classe et un histogramme de quantité d'ADN dans lequel l'intensité de la seconde fluorescence est une classe pour au moins un groupe de particules inclus dans la pluralité de groupes de particules ; et
pour l'au moins un groupe de particules dans lequel l'histogramme de quantité d'ARN et l'histogramme de quantité d'ADN sont créés, la création d'un diagramme bidimensionnel dans lequel l'intensité de la première fluorescence ou l'intensité de la deuxième fluorescence de chaque particule incluse dans le groupe de particules est un axe et la taille de chaque particule incluse dans le groupe de particules est l'autre axe.

2. Procédé d'analyse de particules selon la revendication 1, comprenant en outre l'analyse des particules contenues dans l'échantillon de sang (20) sur la base de l'histogramme de quantité d'ARN et de l'histogramme de quantité d'ADN.

3. Procédé d'analyse de particules selon la revendication 2, comprenant la création de l'histogramme de quantité d'ARN et de l'histogramme de quantité d'ADN pour le groupe d'érythrocytes, dans lequel l'analyse comporte l'un ou plusieurs des éléments suivants :
(a) la mesure du nombre ou rapport de réticulocytes contenus dans l'échantillon de sang (20) et/ou d'une fraction de réticulocytes immatures (IRF) de l'échantillon de sang (20) sur la base de l'histogramme de quantité d'ARN du groupe d'érythrocytes ;
(b) la détermination d'une présence ou absence d'une anomalie dans le groupe d'érythrocytes sur la base de l'histogramme de quantité d'ADN du groupe d'érythrocytes ;
(c) la mesure d'une fraction de plaquettes immatures (IPF) dans l'échantillon de sang (20) sur la base de l'histogramme de quantité d'ARN du groupe de plaquettes ; et
(d) la détermination d'une présence ou absence d'une anomalie dans le groupe de plaquettes sur la base de l'histogramme de quantité d'ADN du groupe de plaquettes.

4. Procédé d'analyse de particules selon la revendication 3, dans lequel l'anomalie est une présence d'un corps, d'un parasite du paludisme, de Babesia, de Theileria, de Trypanosoma, ou d'une microphilie de filaires dans les particules incluses dans le groupe de particules.

5. Procédé d'analyse de particules selon la revendication 1, comprenant en outre la division d'un axe indiquant la taille de chacune des particules dans le diagramme bidimensionnel en une pluralité de régions et la reclassification de l'au moins un groupe de particules en une pluralité de sous-groupes sur la base du nombre ou rapport des particules dans chacune de la pluralité de régions formées par la division ;
éventuellement dans lequel le diagramme bidimensionnel comporte un diagramme bidimensionnel pour un groupe d'érythrocytes, le procédé d'analyse de particules comprenant en outre la reclassification du groupe d'érythrocytes en une pluralité de sous-groupes comportant un sous-groupe d'érythrocytes normaux, un sous-groupe de grands érythrocytes, un sous-groupe d'érythrocytes perturbés, et/ou un sous-groupe de petits érythrocytes.

6. Procédé d'analyse de particules selon la revendication 5, dans lequel le diagramme bidimensionnel comporte un diagramme bidimensionnel pour un groupe de plaquettes, le procédé d'analyse de particules comprenant en outre la reclassification du groupe de plaquettes en une pluralité de sous-groupes comportant un sous-groupe de plaquettes normales, un sous-groupe de plaquettes géantes, un sous-groupe de grandes plaquettes, et/ou un sous-groupe de petites plaquettes.

7. Procédé d'analyse de particules selon l'une quelconque des revendications 5 ou 6, dans lequel le diagramme bidimensionnel comporte un diagramme bidimensionnel pour un groupe de cellules nucléées, le procédé d'analyse de particules comprenant en outre la reclassification du groupe de cellules nucléées en une pluralité de sous-groupes comportant un sous-groupe de cellules nucléées normales, un sous-groupe de grandes cellules nucléées, un sous-groupe de cellules nucléées perturbées, et/ou un sous-groupe de petites cellules nucléées.

8. Procédé d'analyse de particules selon l'une quelconque des revendications 1 à 7, dans lequel la première fluorescence est une fluorescence orange, et la seconde fluorescence est une fluorescence verte, éventuellement dans lequel le colorant métachromatique orthochromatique (10, 10A) est l'orange d'acridine (A0) .

9. Procédé d'analyse de particules selon l'une quelconque des revendications 1 à 7, dans lequel une longueur d'onde centrale de la lumière appliquée aux particules contenues dans l'échantillon de sang (20) est de 408 nm, 445 nm, 473 nm ou 488 nm.

10. Analyseur de particules comprenant :
une source lumineuse (52) qui applique de la lumière aux particules contenues dans un échantillon de sang (20) ;
une cellule de flux (51) à travers laquelle l'échantillon de sang (20) circule ;
un détecteur de lumière comportant une pluralité de détecteurs de fluorescence (61, 62, 63, 64, 65) qui détectent chacun l'intensité d'une première fluorescence et l'intensité d'une seconde fluorescence ayant des longueurs d'onde différentes ; et
une partie de traitement de données (70) qui :
normalise les intensités de la première fluorescence et de la seconde fluorescence émises par chacune des particules contenues dans l'échantillon de sang (20) par la taille de chacune des particules pour déterminer chaque concentration de fluorescence de la première fluorescence et de la seconde fluorescence dans chacune des particules ;
regroupe chacune des particules en une pluralité de groupes de particules comportant au moins deux d'un groupe d'érythrocytes, d'un groupe de plaquettes et d'un groupe de cellules nucléées, dans un graphique bidimensionnel de la concentration de fluorescence obtenue par la normalisation ;
crée un histogramme de quantité d'ARN dans lequel l'intensité de la première fluorescence est une classe et un histogramme de quantité d'ADN dans lequel l'intensité de la seconde fluorescence est une classe pour au moins un groupe de particules inclus dans la pluralité de groupes de particules ; et
pour l'au moins un groupe de particules dans lequel l'histogramme de quantité d'ARN et l'histogramme de quantité d'ADN sont créés, crée un diagramme bidimensionnel dans lequel l'intensité de la première fluorescence ou l'intensité de la deuxième fluorescence de chaque particule incluse dans le groupe de particules est un axe et la taille de chaque particule incluse dans le groupe de particules est l'autre axe.

11. Analyseur de particules selon la revendication 10, dans lequel la partie de traitement de données (70) analyse les particules contenues dans l'échantillon de sang (20) sur la base de l'histogramme de quantité d'ARN et de l'histogramme de quantité d'ADN.
